# EUROPEAN PATENT SPECIFICATION

(11) **EP 2 187 217 B1**
(45) Date of publication and mention of the grant of the patent: **06.11.2013**
(21) Application number: 08828673.7
(22) Date of filing: 22.08.2008
(51) Int. Cl.: C12Q 1/48, C07K 16/40, G01N 33/574

(54) **DETECTION METHOD OF GYNECOLOGIC CANCER**
VERFAHREN ZUR ERKENNUNG VON GYNÄKOLOGISCHEM KREBS
PROCEDE DE DETECTION DE CANCER GYNECOLOGIQUE

(30) Priority: 24.08.2007 JP 2007219046; 13.03.2008 JP 2008063641
(43) Date of publication of application: 19.05.2010
(73) Proprietor: LSIP, LLC, Chiyoda-ku Tokyo 100-0005 (JP)
(72) Inventor: YAMASHITA, Katsuko, Yokohama-shi Kanagawa 226-8503 (JP); SEKO, Akira, Yokohama-shi Kanagawa 226-8503 (JP); AOKI, Daisuke, Tokyo 160-8582 (JP); SAKAMOTO, Masaru, Tokyo 101-0062 (JP)
(74) Representative: Cabinet Plasseraud
(86) International application number: PCT/JP2008/065014
(87) International publication number: WO 2009/028417

(56) References cited:
- EP-A1- 0 445 750
- AOKI DAISUKE ET AL: "Phenotypic alteration of carbohydrate antigens in gynecological malignancy" ACTA HISTOCHEMICA ET CYTOCHEMICA, vol. 28, no. 2, 1995, pages 197-201, XP009136987 ISSN: 0044-5991
- AKIRA KANOH ET AL: "Ectopic expression of N-acetylglucosamine 6-O-sulfotransferase 2 in chemotherapy-resistant ovarian adenocarcinomas" GLYCOCONJUGATE JOURNAL, KLUWER ACADEMIC PUBLISHERS, BO LNKD- DOI:10.1007/S10719-006-6979-6, vol. 23, no. 5-6, 1 July 2006 (2006-07-01) , pages 453-460, XP019396409 ISSN: 1573-4986
- WANG P H ET AL: "Different enzyme activities of sialyltransferases in gynecological cancer cell lines" EUROPEAN JOURNAL OF GYNAECOLOGICAL ONCOLOGY, vol. 23, no. 3, 2002, pages 221-226, XP009136981 ISSN: 0392-2936
- SAITOH EIKO ET AL: "Galactosyltransferase associated with tumor in patients with ovarian cancer: Factors involved in elevation of serum galactosyltransferase." INTERNATIONAL JOURNAL OF ONCOLOGY, vol. 23, no. 2, August 2003 (2003-08), pages 303-310, XP009136984 ISSN: 1019-6439
- JUNKO YOSHIKI,ET AL.: 'High expression of uridine diphosphate-galactose :Lc3Cer beta1-3 galactosyltransferase in human uterine endometrial cancer-derived cells as measured by enzyme-linked immunosorbent assay and thin layer chromatography-immunostaining.' JPN. J.CANCER RES. vol. 88, no. 7, 1997, pages 669 - 677, XP009136982
- E.V.CHANDRASEKARAN: 'Mucin biosynthesis revisited,,the enzymatic transfer of Gal in beta1,3 linkage to the GalNAc moiety of the core structure R1-GlcNAcbeta1,6GalNAcalpha-O-R2' J.BIOL.CHEM. vol. 267, no. 28, 1992, pages 19929 - 19937, XP003026505
- NOBUYASU HAYASHI,ET AL.: 'Association between expression levels of CA19-9 and N-Acetylglucosamine-beta1,3-galactosyltrans ferase5 gene in human pancreatic cancer tissue.' PATHOLOGY vol. 71, no. 1, 2004, pages 26 - 34, XP008125175
- SOICHIRO ISSHIKI,ET AL: 'Cloning,expression,and characterization of a novel UDP-galactose:beta-N-acetylglucosaminebeta1 ,3-galactosyltransferase (betaGal-T5) responsible for synthesis of type 1 chain in colorectal and pancreatic epithelia and tumor cells derived therefrom' J.BIOL.CHEM. vol. 274, no. 18, 1999, pages 12499 - 12507, XP002928039
- LYDIA MARE AND MARCO TRINCHERA: 'Suppression of beta1,3 galactosyltransferase beta3Gal-T5 in cancer cells reduces sialyl-Lewis a and enhances poly N-acetyllactosamines and sialyl-Lewis x on O-glycans' EUR.J.BIOCHEM vol. 271, no. 1, 2004, pages 186 - 194, XP003026506
- AKIRA KANOH,ET AL.: 'Ectopic expression of N-acetylglucosamine 6-O-sulfotransferase 2 in chemotherapy-resistant ovarian adenocarcinomas.' GLYCOCONJUGATE J. vol. 23, no. 5/6, 2006, pages 453 - 460, XP019396409

### SEQUENCE LISTING

<110> Tokyo Institute of Technology
<120> Method of detecting gynecologic cancer
<130> TIT-807
<150> JP 2007-219046
   <151> 2007-08-24
<150> JP 2008-063641
   <151> 2008-03-13
<160> 35
<170> PatentIn version 3.1
<210> 1
   <211> 28
   <212> DNA
   <213> Artificial
<220>
   <223> Forward primer for b3Gal-T5
<400> 1
   tttgtcgaca gtctaaatcc tttcaaag 28
<210> 2
   <211> 26
   <212> DNA
   <213> Artificial
<220>
   <223> Reverse primer for b3Gal-T5
<400> 2
   tttaagcttc agacaggcgg acaatc 26
<210> 3
   <211> 28
   <212> DNA
   <213> Artificial
<220>
   <223> Forward primer for 3Gal-T1
<400> 3
   tttggatcca gtataactcg ccctactt 28
<210> 4
   <211> 25
   <212> DNA
   <213> Artificial
<220>
   <223> Reverse primer for 3Gal-T1
<400> 4
   tttaagcttc ctaacatctg agatg 25
<210> 5
   <211> 27
   <212> DNA
   <213> Artificial
<220>
   <223> Forward primer for 3Gal-T2
<400> 5
   tttggatccc tgccaggcag agctgga 27
<210> 6
   <211> 26
   <212> DNA
   <213> Artificial
<220>
   <223> Reverse primer for 3Gal-T2
<400> 6
   tttaagctta atgtagttta cggtgg 26
<210> 7
   <211> 28
   <212> DNA
   <213> Artificial
<220>
   <223> Forward primer for 3GalNAc-T1
<400> 7
   tttggatcca gccttcccca ctacaatg 28
<210> 8
   <211> 27
   <212> DNA
   <213> Artificial
<220>
   <223> Reverse primer for 3GalNAc-T1
<400> 8
   tttaagctta ataatggcat gtggtgt 27
<210> 9
   <211> 27
   <212> DNA
   <213> Artificial
<220>
   <223> Forward primer for 3Gal-T4
<400> 9
   tttggatcct tgggggagga gctgctg 27
<210> 10
   <211> 27
   <212> DNA
   <213> Artificial
<220>
   <223> Reverse primer for 3Gal-T4
<400> 10
   tttgtcgact cagctctgaa gccaggc 27
<210> 11
   <211> 27
   <212> DNA
   <213> Artificial
<220>
   <223> Forward primer for 3Gal-T6
<400> 11
   tttggatccg accccagggc gatgtcg 27
<210> 12
   <211> 26
   <212> DNA
   <213> Artificial
<220>
   <223> Reverse primer for 3Gal-T6
<400> 12
   tttaagcttg gctcagggga tgccct 26
<210> 13
   <211> 22
   <212> DNA
   <213> Artificial
<220>
   <223> Forward primer for PCR of 3Gal-T4
<400> 13
   attcttgctg ggagagccga ac 22
<210> 14
   <211> 23
   <212> DNA
   <213> Artificial
<220>
   <223> Reverse primer for PCR of 3Gal-T4
<400> 14
   aagtacagaa gaggcacttc ctc 23
<210> 15
   <211> 22
   <212> DNA
   <213> Artificial
<220>
   <223> Forward primer for PCR of 3Gal-T5
<400> 15
   ggaaacgaaa gaggtggacc ag 22
<210> 16
   <211> 23
   <212> DNA
   <213> Artificial
<220>
   <223> Reverse primer for PCR of 3Gal-T5
<400> 16
   gagctcctcc aatctgatgt tca 23
<210> 17
   <211> 25
   <212> DNA
   <213> Artificial
<220>
   <223> Forward primer for GlcNAc6ST2
<400> 17
   tttgtcgaca gccacaacat cagct 25
<210> 18
   <211> 24
   <212> DNA
   <213> Artificial
<220>
   <223> Reverse primer for GlcNAc6ST2
<400> 18
   tttaagctta gtggatttgc tcag 24
<210> 19
   <211> 26
   <212> DNA
   <213> Artificial
<220>
   <223> Forward primer for GlcNAc6ST2-pichia
<400> 19
   tttcctaggt acagccacaa catcag 26
<210> 20
   <211> 29
   <212> DNA
   <213> Artificial
<220>
   <223> Reverse primer for GlcNAc6ST2-pichia
<400> 20
   tttgcggccg cttagtggat ttgctcagg 29
<210> 21
   <211> 24
   <212> DNA
   <213> Artificial
<220>
   <223> Forward primer for GlcNAc6ST1
<400> 21
   tttgtcgact acaagtggca caag 24
<210> 22
   <211> 24
   <212> DNA
   <213> Artificial
<220>
   <223> Reverse primer for GlcNAc6ST1
<400> 22
   tttgtcgacc ccttttagag acgg 24
<210> 23
   <211> 26
   <212> DNA
   <213> Artificial
<220>
   <223> Forward primer for GlcNAc6ST3
<400> 23
   tttgtcgact cccggccagg gccctc 26
<210> 24
   <211> 26
   <212> DNA
   <213> Artificial
<220>
   <223> Reverse primer for GlcNAc6ST3
<400> 24
   tttaagcttc agtcaggcga tgccca 26
<210> 25
   <211> 23
   <212> DNA
   <213> Artificial
<220>
   <223> Forward primer for GlcNAc6ST4
<400> 25
   tttgtcgacg gcggccgcga cgg 23
<210> 26
   <211> 26
   <212> DNA
   <213> Artificial
<220>
   <223> Reverse primer for GlcNAc6ST4
<400> 26
   tttaagcttg ggaggctacg tggcgc 26
<210> 27
   <211> 26
   <212> DNA
   <213> Artificial
<220>
   <223> Forward primer for GlcNAc6ST5
<400> 27
   tttgtcgact cccggccagg gccctc 26
<210> 28
   <211> 26
   <212> DNA
   <213> Artificial
<220>
   <223> Reverse primer for GlcNAc6ST5
<400> 28
   tttaagcttg ctacaactgt ggcctc 26
<210> 29
   <211> 26
   <212> DNA
   <213> Artificial
<220>
   <223> Forward primer for Gal6ST
<400> 29
   tttgtcgaca ccttcaccgc caagtc 26
<210> 30
   <211> 26
   <212> DNA
   <213> Artificial
<220>
   <223> Reverse primer for Gal6ST
<400> 30
   tttaagcttc acgagaaggg gcggaa 26
<210> 31
   <211> 26
   <212> DNA
   <213> Artificial
<220>
   <223> Forward primer for Ch6ST1
<400> 31
   tttgtcgaca tatcaagggt ctcaga 26
<210> 32
   <211> 27
   <212> DNA
   <213> Artificial
<220>
   <223> Reverse primer for Ch6ST1
<400> 32
   tttaagcttc tacgtgaccc agaaggt 27
<210> 33
   <211> 310
   <212> PRT
   <213> Homo sapiens
<400> 33
   Met Ala Phe Pro Lys Met Arg Leu Met Tyr Ile Cys Leu Leu Val Leu
   1 5 10 15
   Gly Ala Leu Cys Leu Tyr Phe Ser Met Tyr Ser Leu Asn Pro Phe Lys
   20 25 30
   Glu Gln Ser Phe Val Tyr Lys Lys Asp Gly Asn Phe Leu Lys Leu Pro
   35 40 45
   Asp Thr Asp Cys Arg Gln Thr Pro Pro Phe Leu Val Leu Leu Val Thr
   50 55 60
   Ser Ser His Lys Gln Leu Ala Glu Arg Met Ala Ile Arg Gln Thr Trp
   65 70 75 80
   Gly Lys Glu Arg Met Val Lys Gly Lys Gln Leu Lys Thr Phe Phe Leu
   85 90 95
   Leu Gly Thr Thr Ser Ser Ala Ala Glu Thr Lys Glu Val Asp Gln Glu
   100 105 110
   Ser Gln Arg His Gly Asp Ile Ile Gln Lys Asp Phe Leu Asp Val Tyr
   115 120 125
   Tyr Asn Leu Thr Leu Lys Thr Met Met Gly Ile Glu Trp Val His Arg
   130 135 140
   Phe Cys Pro Gln Ala Ala Phe Val Met Lys Thr Asp Ser Asp Met Phe
   145 150 155 160
   Ile Asn Val Asp Tyr Leu Thr Glu Leu Leu Leu Lys Lys Asn Arg Thr
   165 170 175
   Thr Arg Phe Phe Thr Gly Phe Leu Lys Leu Asn Glu Phe Pro Ile Arg
   180 185 190
   Gln Pro Phe Ser Lys Trp Phe Val Ser Lys Ser Glu Tyr Pro Trp Asp
   195 200 205
   Arg Tyr Pro Pro Phe Cys Ser Gly Thr Gly Tyr Val Phe Ser Gly Asp
   210 215 220
   Val Ala Ser Gln Val Tyr Asn Val Ser Lys Ser Val Pro Tyr Ile Lys
   225 230 235 240
   Leu Glu Asp Val Phe Val Gly Leu Cys Leu Glu Arg Leu Asn Ile Arg
   245 250 255
   Leu Glu Glu Leu His Ser Gln Pro Thr Phe Phe Pro Gly Gly Leu Arg
   260 265 270
   Phe Ser Val Cys Leu Phe Arg Arg Ile Val Ala Cys His Phe Ile Lys
           275                 280                 285
   Pro Arg Thr Leu Leu Asp Tyr Trp Gln Ala Leu Glu Asn Ser Arg Gly
       290                 295                 300
   Glu Asp Cys Pro Pro Val
   305                 310
<210> 34
   <211> 378
   <212> PRT
   <213> Homo sapiens
<400> 34
   Met Gln Leu Arg Leu Phe Arg Arg Leu Leu Leu Ala Ala Leu Leu Leu
   1               5                   10                  15
   Val Ile Val Trp Thr Leu Phe Gly Pro Ser Gly Leu Gly Glu Glu Leu
               20                  25                  30
   Leu Ser Leu Ser Leu Ala Ser Leu Leu Pro Ala Pro Ala Ser Pro Gly
           35                  40                  45
   Pro Pro Leu Ala Leu Pro Arg Leu Leu Ile Pro Asn Gln Glu Ala Cys
       50                  55                  60
   Ser Gly Pro Gly Ala Pro Pro Phe Leu Leu Ile Leu Val Cys Thr Ala
   65                  70                  75                  80
   Pro Glu Asn Leu Asn Gln Arg Asn Ala Ile Arg Ala Ser Trp Gly Gly
                   85                  90                  95
   Leu Arg Glu Ala Arg Gly Leu Arg Val Gln Thr Leu Phe Leu Leu Gly
               100                 105                 110
   Glu Pro Asn Ala Gln His Pro Val Trp Gly Ser Gln Gly Ser Asp Leu
           115                 120                 125
   Ala Ser Glu Ser Ala Ala Gln Gly Asp Ile Leu Gln Ala Ala Phe Gln
       130                 135                 140
   Asp Ser Tyr Arg Asn Leu Thr Leu Lys Thr Leu Ser Gly Leu Asn Trp
   145                 150                 155                 160
   Ala Glu Lys His Cys Pro Met Ala Arg Tyr Val Leu Lys Thr Asp Asp
                   165                 170                 175
   Asp Val Tyr Val Asn Val Pro Glu Leu Val Ser Glu Leu Val Leu Arg
               180                 185                 190
   Gly Gly Arg Trp Gly Gln Trp Glu Arg Ser Thr Glu Pro Gln Arg Glu
           195                 200                 205
   Ala Glu Gln Glu Gly Gly Gln Val Leu His Ser Glu Glu Val Pro Leu
       210                 215                 220
   Leu Tyr Leu Gly Arg Val His Trp Arg Val Asn Pro Ser Arg Thr Pro
   225                 230                 235                 240
   Gly Gly Arg His Arg Val Ser Glu Glu Gln Trp Pro His Thr Trp Gly
                   245                 250                 255
   Pro Phe Pro Pro Tyr Ala Ser Gly Thr Gly Tyr Val Leu Ser Ala Ser
               260                 265                 270
   Ala Val Gln Leu Ile Leu Lys Val Ala Ser Arg Ala Pro Leu Leu Pro
           275                 280                 285
   Leu Glu Asp Val Phe Val Gly Val Ser Ala Arg Arg Gly Gly Leu Ala
       290                 295                 300
   Pro Thr Gln Cys Val Lys Leu Ala Gly Ala Thr His Tyr Pro Leu Asp
   305                 310                 315                 320
   Arg Cys Cys Tyr Gly Lys Phe Leu Leu Thr Ser His Arg Leu Asp Pro
                   325                 330                 335
   Trp Lys Met Gln Glu Ala Trp Lys Leu Val Gly Gly Ser Asp Gly Glu
               340                 345                 350
   Arg Thr Ala Pro Phe Cys Ser Trp Phe Gln Gly Val Leu Gly Ile Leu
           355                 360                 365
   Arg Cys Arg Ala Ile Ala Trp Leu Gln Ser
       370                 375
<210> 35
   <211> 386
   <212> PRT
   <213> Homo sapiens
<400> 35
   Met Leu Leu Pro Lys Lys Met Lys Leu Leu Leu Phe Leu Val Ser Gln
   1               5                   10                  15
   Met Ala Ile Leu Ala Leu Phe Phe His Met Tyr Ser His Asn Ile Ser
               20                  25                  30
   Ser Leu Ser Met Lys Ala Gln Pro Glu Arg Met His Val Leu Val Leu
           35                  40                  45
   Ser Ser Trp Arg Ser Gly Ser Ser Phe Val Gly Gln Leu Phe Gly Gln
               50                  55                  60
   His Pro Asp Val Phe Tyr Leu Met Glu Pro Ala Trp His Val Trp Met
   65                  70                  75                  80
   Thr Phe Lys Gln Ser Thr Ala Trp Met Leu His Met Ala Val Arg Asp
                   85                  90                  95
   Leu Ile Arg Ala Val Phe Leu Cys Asp Met Ser Val Phe Asp Ala Tyr
               100                 105                 110
   Met Glu Pro Gly Pro Arg Arg Gln Ser Ser Leu Phe Gln Trp Glu Asn
           115                 120                 125
   Ser Arg Ala Leu Cys Ser Ala Pro Ala Cys Asp Ile Ile Pro Gln Asp
       130                 135                 140
   Glu Ile Ile Pro Arg Ala His Cys Arg Leu Leu Cys Ser Gln Gln Pro
   145                 150                 155                 160
   Phe Glu Val Val Glu Lys Ala Cys Arg Ser Tyr Ser His Val Val Leu
                   165                 170                 175
   Lys Glu Val Arg Phe Phe Asn Leu Gln Ser Leu Tyr Pro Leu Leu Lys
               180                 185                 190
   Asp Pro Ser Leu Asn Leu His Ile Val His Leu Val Arg Asp Pro Arg
           195                 200                 205
   Ala Val Phe Arg Ser Arg Glu Arg Thr Lys Gly Asp Leu Met Ile Asp
       210                 215                 220
   Ser Arg Ile Val Met Gly Gln His Glu Gln Lys Leu Lys Lys Glu Asp
   225                 230                 235                 240
   Gln Pro Tyr Tyr Val Met Gln Val Ile Cys Gln Ser Gln Leu Glu Ile
                   245                 250                 255
   Tyr Lys Thr Ile Gln Ser Leu Pro Lys Ala Leu Gln Glu Arg Tyr Leu
               260                 265                 270
   Leu Val Arg Tyr Glu Asp Leu Ala Arg Ala Pro Val Ala Gln Thr Ser
           275                 280                 285
   Arg Met Tyr Glu Phe Val Gly Leu Glu Phe Leu Pro His Leu Gln Thr
       290                 295                 300
   Trp Val His Asn Ile Thr Arg Gly Lys Gly Met Gly Asp His Ala Phe
   305                 310                 315                 320
   His Thr Asn Ala Arg Asp Ala Leu Asn Val Ser Gln Ala Trp Arg Trp
                   325                 330                 335
   Ser Leu Pro Tyr Glu Lys Val Ser Arg Leu Gln Lys Ala Cys Gly Asp
               340                 345                 350
   Ala Met Asn Leu Leu Gly Tyr Arg His Val Arg Ser Glu Gln Glu Gln
           355                 360                 365
   Arg Asn Leu Leu Leu Asp Leu Leu Ser Thr Trp Thr Val Pro Glu Gln
       370                 375                 380
   Ile His
   385

## Description

### TECHNICAL FIELD

The present invention relates to a method for detecting ovarian cancer or endometrial cancer, characterized in that β1,3-galactosyltransferase-5 and/or β1,3-galactosyltransferase-4, and GlcNAc 6-O-sulfotransferase are analyzed. The term "analyzing" or "analysis" as used herein includes a quantitative or semiquantitative measurement of an amount of a compound to be analyzed and a detection used to determine the presence or the absence of a compound to be analyzed.

### BACKGROUND ART

A gynecologic cancer includes, for example, endocervical cancer, endometrial cancer, ovarian cancer, valvar cancer, vaginal cancer, uterine sarcoma, and chorionic cancer (trophoblastic disease). In these many cancers, it is difficult to find the cancer by symptoms thereof at an early stage. However, five years survival rate is significantly increased by carrying out a surgical therapy such as operation at an early stage. Thus, it is desired to develop tumor marker capable of finding the gynecologic cancer at an early stage.
Ovarian cancer is developed in an ovary within the abdomen. Thus, even if the cancer has developed in patients, the patients with cancer hardly have subjective symptoms at an early stage. Therefore, in many cases, ovarian cancer patients are found in advanced stages such as stage III or stage IV after metastasizing. In such patients, even if a treatment is carried out, five years survival rate becomes low. For the treatment to the ovarian cancer patients, surgical therapy to operatively remove an ovary and a caul, irradiation therapy, and chemotherapy using anticancer drug are carried out.
At a less-advanced stage such as stage I or stage II in the ovarian cancer patients, it may be possible to cure ovarian cancer completely by operatively removing an ovary and surrounding tissues. On the contrary, at an advanced stage such as stage III or stage IV, metastasis of the ovarian cancer to the lymph node or the liver is developed. Since cancer tissue cannot be removed completely from the patients, irradiation therapy or chemotherapy is therefore carried out concurrently with the surgical therapy. However, it is difficult to completely prevent the metastatic cancer by irradiation therapy and chemotherapy, and thus, five years survival rate of such patients is extremely low in comparison with those of the patients which are found at stage I or II. Therefore, findings of the ovarian cancer patients at an early stage are desired.

As mentioned above, the ovarian cancer patients hardly have subjective symptoms at an early stage, and therefore many of the patients are found by means of diagnostic imaging technique using ultrasonic sound at an advanced stage.
As a diagnostic marker for detecting ovarian cancer, a measurement of glycoprotein, i.e. CA125, is most widely used. High positive rate of CA125 in serous adenocarcinoma is well known among four histological types of ovarian cancer, i.e. serous adenocarcinoma, mucinous adenocarcinoma, endometrioid adenocarcinoma, and clear cell adenocarcinoma. In addition, CA125 is a specific marker for ovarian cancer. Many of patients at advanced stage have positive CA125. However, positive rate of CA125 in the ovarian cancer patients at an early stage is low. Although an attempt of early diagnosis by means of CA125 assay in a health examination has been made in Europe and the United states, effectiveness thereof could not be revealed.

Endometrial cancer is developed in an endometrium within the uterus, and cannot be detected by cytodiagnosis which is carried out in a conventional gynecologic examination. The endometrial cancer patients are treated with surgical therapy by operatively removing cancer tissue, irradiation therapy, chemotherapy using anticancer drug, and hormonal therapy.
Stages of endometrial cancer may be classified into stage I to stage IV in accordance with the spread of cancer. Surgical therapy is applied in stages I to III, and then five years survival rate has been increased by carrying out the surgical therapy at an early stage. Therefore, findings of the endometrial cancer patients at an early stage are desired.

As a diagnostic marker of the endometrial cancer, CA125 or CA602 which is glycoprotein, or CA19-9 which is used as diagnostic marker for the pancreas cancer is used. However, the detection rates of the endometrial cancer patients by the diagnostic markers are extremely low, and thus, these diagnostic markers have no diagnostic benefit.

In various cancer cells and cancer tissues, increase or decrease of many proteins are reported. For example, it was reported that β1,3-galactosyltransferase-5 (hereinafter referred to as a β3Gal-T5), which is a glycosyltransferase involved in the synthesis of typel(Galβ1-3GlcNAc) is expressed in colon cancer cells and pancreas cancer cells, and colon cancer tissues (patent reference 1 and non-patent reference 1). In addition, it has been reported that GlcNAc 6-O-sulfotransferase-2 (hereinafter referred to as a GlcNAc6ST-2), which is a sulfotransferase involved in the synthesis of L-selectin ligand, is expressed ectopically in mucinous colon cancer tissues (non-patent reference 2), and tissues of mucinous adenocarcinoma and clear cell adenocarcinoma (non-patent reference 3).

However, these reports merely disclose that the glycosyl/sulfotransferases are expressed in cancer cells or cancer tissues, but do not disclose usefulness of the glycosyl/sulfotransferases as a tumor marker in blood. Glycosyltransferases are not generally secreted to the outside of cells, and thus, it was thought that these glycosyltransferases are not useful as the tumor marker in bloods. Further, there is no report showing a correlation between β3Gal-T5 and ovarian cancer, β3Gal-T5 and endometrial cancer, or GlcNAc6ST-2 and endometrial cancer. Furthermore, as to β1,3-galactosyltransferase-4 (hereinafter referred to as a β3Gal-T4) which is one of β1,3-glycosyltransferase family, a correlation between β3Gal-T4 and ovarian cancer or endometrial cancer has not been reported.

### CITATION LIST

[patent reference 1] International Publication WO00/50608 [non-patent reference 1] Seko A., et. al. Cancer Research, 1996, (the United state), vol. 56, p.3468-3473
[non-patent reference 2] Seko A., et al. Glycobiology, 2002, (the United state) vol.12, p.379-388
[non-patent reference 3] Kanoh A., et al. Glycoconjugate Journal, 2006, (Netherlands) vol.23, p.453-460

### DISCLOSURE OF THE INVENTION

### PROBLEMS TO BE SOLVED BY THE INVENTION

The present inventors have conducted intensive studies for a possibility of various intracellular proteins as a tumor marker, to find a detective marker capable of detecting ovarian cancer patients and endometrial cancer patients. As a result, the present inventors, surprisingly, found that β3Gal-T4 and/or β3Ga1-T5, or GlcNAc6ST-2 were detected in the blood of the ovarian cancer patients at high rates. Particularly, the positive rate of β3Gal-T4 and/or β3Gal-T5 was high in all histological types of ovarian cancer, i.e. serous adenocarcinoma, mucinous adenocarcinoma, endometrioid adenocarcinoma, and clear cell adenocarcinoma. Furthermore, a scope of cancer cells secreting β3Gal-T4 and/or β3Gal-T5 does not fully overlap with that of cancer cells secreting GlcNAc6ST-2. Therefore, the present inventors found that a detective rate of ovarian cancer becomes significantly higher by a combination of an assay of β3Gal-T4 and/or β3Gal-T5, and an assay of GlcNAc6ST-2.
Further, the present inventors found that β3Gal-T4 and/or β3Gal-T5, or GlcNAc6ST-2 were detected in the blood of the endometrial cancer patients at high rates. In particular, the high positive rate of β3Gal-T4 and/or β3gal-T5 in endometrial cancer patients is shown in comparison with CA125, CA602, or CA19-9. Furthermore, a scope of cancer cells secreting β3Gal-T4 and/or β3Gal-T5 does not fully overlap with one of cancer cells secreting GlcNAc6ST-2. Therefore, the present inventors found that a detective rate of endometrial cancer becomes significantly higher by the combination of the assay of β3Gal-T4 and/or β3Gal-T5. and the assay of GlcNAc6ST-2, and completed the present invention.
In this connection, the non-patent reference 3 discloses that GlcNAc6ST-2 is expressed ectopically in tissues of serous adenocarcinoma and clear cell adenocarcinoma. However, the non-patent reference 3 discloses in addition to the above information, that an expression of GlcNAc6ST-2 was not detected in endometrioid adenocarcinoma. That is, the non-patent reference 3 merely discloses that GlcNAc6ST-2 is expressed in tissues of serous adenocarcinoma and clear cell adenocarcinoma, and therefore the high detection rate of GlcNAc6ST-2 in blood of ovarian cancer patients could not be anticipated. In other words, it is unexpected for those skilled in the art that GlcNAc6ST-2 can be detected in blood of the patients with serous adenocarcinoma and clear cell adenocarcinoma at high rates, despite the fact that GlcNAc6ST-2 could not be detected in the tissues of serous adenocarcinoma and clear cell adenocarcinoma.

Accordingly, the object of the present invention is to provide a method for detecting ovarian cancer or endometrial cancer, and a kit for detecting the same, and a use of a kit for detecting the same.

### MEANS FOR SOLVING THE PROBLEMS

The present invention relates to methods for detecting ovarian cancer or endometrial cancer as defined in the claims characterized in that β1,3-galactosyltransferase-5 and/or β1,3-galactosyltransferase-4, GlcNAc 6-O-sulfotransferase-2, respectively, are analyzed.
According to a preferable embodiment of the detecting method of the present invention, an antibody specifically binding to β1,3-galactosyltransferase-5 and β1,3-galactosyltransferase-4 is used. According to a preferable embodiment of the detecting method of the present invention, an antibody specifically binding to GlcNAc 6-O-sulfotransferase-2 is used.
As a sample to be tested in the method for detecting of the present invention, a biological sample isolated or derived from human body, for example, urine, blood, serum, plasma, spinal fluid, saliva, cell, tissue, organ, or preparation thereof (for example, biopsy specimen, particularly biopsy specimen of ovary) or the like may be used. However the samples to be used are specified as defined in the claims. The detecting method of the present invention can be used as a diagnostic method of ovarian cancer or endometrial cancer, by detecting β1,3-galactosyltransferase-5 and/or β1,3-galactosyltransferase-4, and GlcNAc 6-O-sulfotransferase-2, respectively, from the samples to be tested isolated from human body, as specified in the claims.
Further, the present invention relates to a kit for detecting ovarian cancer or endometrial cancer characterized by containing an antibody or a fragment thereof specifically binding to β1,3-galactosyltransferase-5 and β1,3-galactosyltransferase-4, and an antibody or a fragment thereof specifically binding to GlcNAc 6-O-sulfotransferase-2.
Further, the present invention relates to a use of a kit for detecting ovarian or endometrial cancer characterized by containing an antibody or a fragment thereof specifically binding to β1,3-galactosyltransferase-5 and βl,3-galactosyltransferase-4.
Further, the present invention relates to a use of a kit for detecting ovarian or endometrial cancer characterized by containing an antibody or a fragment thereof specifically binding to GlcNAc 6-O-sulfotransferase-2.

The present invention relates to a method for detecting ovarian cancer characterized in that β1,3-galactosyltransferase-5 and/or α1,3-galactosyltransferase-4, and GlcNAc 6-O-sulfotransferase-2 are analyzed.
Further, the present invention relates to a method for detecting ovarian cancer characterized in that β1,3-galactosyltransferase-5 and/or β1,3- galactosyltransferase-4 are analyzed.
Further, the present invention relates to a method for detecting ovarian cancer characterized in that GlcNAc 6-O-sulfotransferase in blood, serum or plasma is analyzed.
According to a preferable embodiment of the detecting method of the present invention, an antibody specifically binding to β1,3-galactosyltransferase-5 and β1,3-galactosyltransferase-4 is used.
According to a preferable embodiment of the detecting method of the present invention, an antibody specifically binding to GlcNAc 6-O-sulfotransferase-2 is used.
Further, the present invention relates to a kit for detecting ovarian cancer characterized by containing an antibody or a fragment thereof specifically binding to β1,3-galactosyltransferase-5 and β1,3-galactosyltransferase-4 thereof, and an antibody or a fragment thereof specifically binding to GlcNAc 6-O-sulfotransferase-2.
Further, the present invention relates to a use of a kit for detecting ovarian cancer characterized by containing an antibody or a fragment thereof specifically binding to β1,3-galactosyltransferase-5 and β1,3-galactosyltransferase-4.
Further, the present invention relates to a use of a kit for detecting ovarian cancer by analyzing GlcNAc 6-O-sulfotransferase in blood, serum or plasma characterized by containing an antibody or a fragment thereof specifically binding to GlcNAc 6-O-sulfotransferase-2.
The invention also relates to methods for detecting endometrial cancer, uses of kit for detecting said cancer and a kit for detecting said cancer as defined in claims 2, 4, 6, 7, 8, 10, 12 and 14.

### EFFECTS OF THE INVENTION

Hitherto, as a detective marker of ovarian cancer, CA125 or CA546 which are glycoproteins was widely used. According to the detecting method of the present invention, ovarian cancer patients can be detected at high rates in early stages i.e. stage I or II, in comparison with the above conventional detective marker of ovarian cancer. That is, according to the detecting method of the present invention, β3Gal-T4 and/or β3Gal-T5, or GlcNAc6ST detected in blood, serum or plasma, which are secreted from relatively-small cancer tissue, can also be detected, and therefore, ovarian cancer patients of stage I, who have no subjective symptoms, can be detected in a health examination. In addition, according to the detecting method wherein β3Gal-T4 and/or β3Gal-T5 is analyzed, of the present invention, mucinous adenocarcinoma, endometrioid adenocarcinoma, and clear cell adenocarcinoma can be detected in high rate in comparison with the conventional method, in particular, mucinous adenocarcinoma and clear cell adenocarcinoma, which are intractable and resistive to the chemotherapy, can be detected at high rates.
A detective rate of ovarian cancer can be significantly increased by a combination of a detecting method wherein β3Gal-T4 and/or β3Gal-T5 is analyzed of the present invention, and a detecting method wherein GlcNAc6ST-2 is analyzed of the present invention. In particular, in the patients at an early stage i.e. stage I or II of ovarian cancer, the combination thereof is significantly effective.
In addition, in the patients of histological types of endometrioid adenocarcinoma and clear cell adenocarcinoma, the detection rate of ovarian cancer can be increased by the combination thereof.
As a detective marker of endometrial cancer, CA125, CA602, or CA19-9 was used. According to the detecting method of the present invention, endometrial cancer can be detected at high rates at an early stage i.e. stage I, in comparison with the above conventional detective markers. That is, according to the detecting method of the present invention, β3Gal-T4 and/or β3Gal-T5, or GlcNAc6ST-2, which are secreted from relatively-small amount of cancer tissue, can also be detected in blood, serum or plasma. Therefore, endometrial cancer, which may not be detected by cytodiagnosis in conventional gynecologic examination, can be detected.
Further, the detection rate of endometrial cancer can be significantly increased by the combination of a detecting method wherein β3Gal-T4 and/or β3Gal-T5 is analyzed of the present invention, and a detecting method wherein GlcNAc6ST-2 is analyzed of the present invention.
Furthermore, the detective method of the present invention can be also used in monitoring for a recurrence of cancer, and monitoring effects of irradiation therapy and chemotherapy.

### BRIEF DESCRIPTION OF DRAWINGS

[Fig. 1]

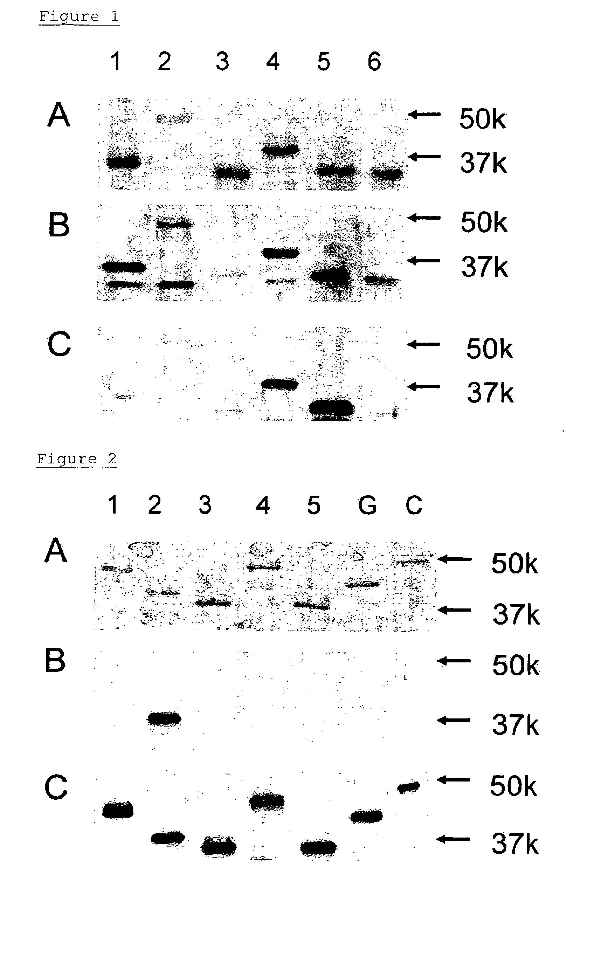

   Figure 1 is photographs showing the specificity of monoclonal antibody and polyclonal antisera against 3Gal-T5. A: β3GalT-1 (lane 1), β3GalT-2 (lane 2), β3GalNAc-T1 (lane 3), β3Gal-T4 (lane 4), β3Gal-T5 (lane 5), and β3GalT-6 (lane 6) were subjected to electrophoresis, and stained by Sypro Orange (Invitrogen). B: Each protein was subjected to a western blotting method using a monoclonal antibody against β3Gal-T5. C: Each protein was subjected to a western blotting method using a polyclonal antisera against β3Gal-T5.
[Fig. 2]
   Figure 2 is photographs showing the specificity of monoclonal antibody and polyclonal antisera against GlcNAc6ST-2. A: GlcNAc6STl (lane 1), GlcNAc6ST-2 (lane 2), GlcNAc6ST-3 (lane 3), GlcNAc6ST-4 (lane 4), GlcNAc6ST-5 (lane 5), Gal6ST (lane G), and Ch6ST1 (lane C) were subjected to electrophoresis, and stained by Sypro Orange (Invitrogen). B: Each protein was subjected to a Western blotting method using a monoclonal antibody against GlcNAc6ST-2. C: Each protein was subjected to Western blotting method using a polyclonal antisera against GlcNAc6ST-2.
[Fig. 3]
   Figure 3A is a standard curve of β3Gal-T5 by a sandwich ELISA using a monoclonal antibody and polyclonal antisera against β3Ga1-T5. Figure 3B is a standard curve of GlcNAc6ST-2 by a sandwich ELISA using a monoclonal antibody and polyclonal antisera against GlcNAc6ST-2.
   [Fig. 4] Figure 4 is photographs wherein expression of mRNAs of β3Gal-T4 and β3Gal-T5 in five ovarian cancer cell lines i.e. ES-2 (lane 1), MCAS (lane 2), RMG-I (lane 3), RMG-II (lane 4), and RMUG-L (lane 5), were shown by RT-PCR.
[Fig. 5]

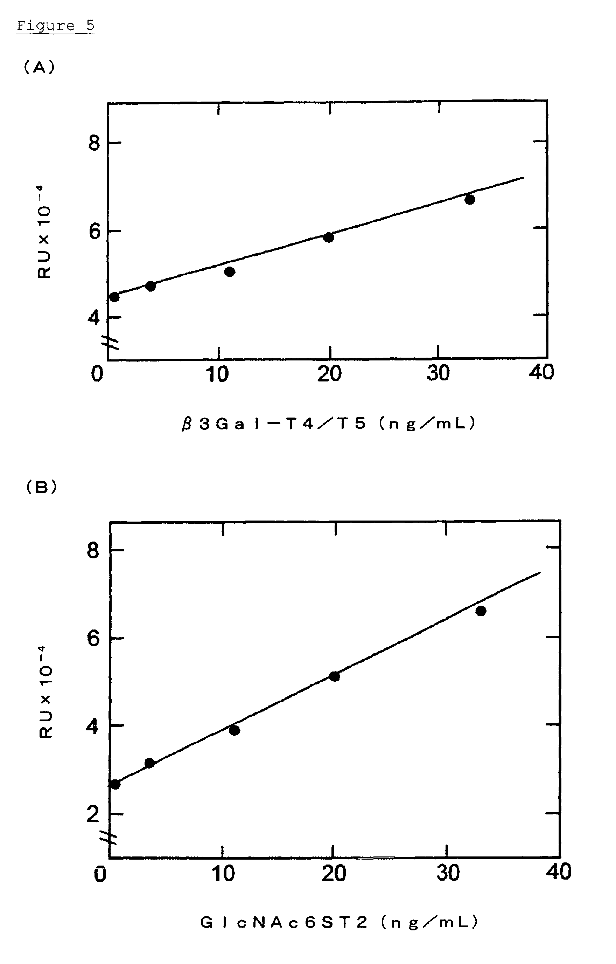

   Figure 5A is a standard curve of β3Gal-T5 by a sandwich ELISA using a monoclonal antibody and polyclonal antisera against β3Gal-T5. As a diluent for standard substance, non-denatured β3Gal-T5 (E.coli), PBS with 0.1% Tween-20 (PBS-T) including 10% normal human serum was used. Figure 5B is a standard curve of GlcNAc6ST-2 by a sandwich ELISA using a monoclonal antibody and polyclonal antisera against GlcNAc6ST-2. As a diluent for standard substance, non-denatured GlcNAc6ST-2 (E.coli), PBS with 0.1% Tween-20 (PBS-T) including 10% normal human serum was used.

### BEST MODE FOR CARRYING OUT THE INVENTION

### [1] A method for detecting ovarian and endometrial cancers

According to the detecting method of the present invention, these cancers can be detected in patients. Particularly, ovarian cancer and endometrial cancer are detected with a high detection rate. Therefore, as embodiments of the present invention, ovarian cancer and endometrial cancer are described hereinafter.

### [1-1] Ovarian cancer

The ovary is an oval organ about the size of thumb located at both sides of the uterus. Ovarian cancer develops therein, and is mostly an epithelial cancer. Ovarian cancer can be classified into stages I to IV, according to the cancer progression, as described bellow. Stage I: The cancer is only inside the ovary. That is, the cancer is in one or both ovaries and is either on the outside of an ovary
Stage II: The cancer is in one or both ovaries and is extending into the pelvic tissues. That is, the cancer has spread (metastasized) to around an ovary (for example peritoneum such as the fallopian tubes, uterus, the rectum, or the bladder) within the pelvis.
Stage III: The cancer has spread (metastasized) (1) beyond the pelvis with peritoneal dissemination; (2) the retroperitoneum lymph nodes or inguinal lymph nodes; (3) the surface of liver; (4) the small intestine or colon histlogically. That is, the cancer has not been limited to around an ovary (peritoneum in the pelvic), but has spread (metastasized) to upper abdomen, or to retroperitoneum lymph nodes.
Stage IV: (1) the cancer has spread (metastasized) to the distant site; (2) the cancer cells are found in pleural fluid; (3) the cancer has spread (metastasized) to the inside of the liver. That is, the cancer has spread to the outside of the peritoneal cavity or the inside of the liver.

Due to an absence of subjective symptoms at an early stage, only about a third of ovarian cancer cases are found in stages I and II, and thus, two thirds thereof are found in stages III and IV. When ovarian cancer is found in stages III and IV, metastasis of ovarian cancer to an outside of cavitas pelvis has occurred.
Therefore, complete cure is difficult because the ovarian cancer found cannot be removed fully.

Although the histological types of ovarian cancer are varied, ovarian cancer may be classified mainly into serous adenocarcinoma, mucinous adenocarcinoma, endometrioid adenocarcinoma, and clear cell adenocarcinoma. Among the four histological types, it is considered that serous adenocarcinoma occurs most frequently. In most of the conventional ovarian cancer markers, such serous adenocarcinoma is detected. Further, in view of sensitivity to chemotherapy, it is considered that serous adenocarcinoma and clear cell adenocarcinoma exhibit a high resistance to anti tumor drug.

### [1-2] Endometrial cancer

Endometrial cancer is developed in epithelium of uterus i.e. endometrium, and thus, is an epithelial cancer. Endometrial cancer can be classified into stages I to IV, according to the stage of cancer progression, as described bellow.
Stage I: The cancer is found in the uterine corpus only. That is, the cancer is not found in the uterine cervix or other parts.
Stage II: The cancer has spread from the uterus to the cervix. That is the cancer has not spread outside the uterus.
Stage III: the cancer has spread beyond the uterus and cervix, but has not spread beyond the pelvis. The cancer has spread (metastasized) to pelvic lymph nodes or periaortic lymph nodes.
Stage IV: The cancer has spread to other parts of the body beyond the pelvis; or bladder or bowel wall.

Endometrial cancer cannot be found by the cytodiagnosis for endocervical cancer in the conventional gynecologic examination. However, if the endometrial cancer cases can be found in stages 1 to III, surgical therapy by operation is applicable. Further, if the operation is carried out at an earlier stage, effect of therapy becomes higher.

### [1-3] Analysis of β3Gal-T4 and/or β3Gal-T5

In the present invention, ovarian cancer or endometrial cancer is detected by analyzing β3Gal-T4 and/or β3Gal-T5. Also, gynecologic cancer, in particular, ovarian cancer or endometrial cancer can be detected by analyzing β3Ga1-T4. In addition, ovarian cancer or endometrial cancer can be also detected by analyzing β3Gal-T5. Further, ovarian cancer or endometrial cancer can be also detected by analyzing β3Gal-T4 and β3Gal-T5, all as specified in the claims.

β3Gal-T4 belongs to β1,3-galactosyltransferase family, and synthesizes GDla ganglioside, GM1 ganglioside, or GDlb ganglioside. While β3Gal-T5 exhibits a different substrate specificity from that of β3Gal-T4, and can synthesize Galβ1-3 GlcNAc structure.

As shown in Example 2, mRNAs of β3Gal-T4 and β3Gal-T5 are expressed in most cell lines derived from ovarian cancer. The scope of cell lines expressing mRNA of β3Gal-T4 does not fully overlap with that of β3Gal-T5. However, in all examined cell lines, mRNAs of β3Gal-T4 and/or β3Gal-T5 are expressed. In addition, the present inventors measured enzymatic activities of β3Gal-T4 and β3Gal-T5 in ovarian cancer tissues of five patients, and could detect the enzymatic activities of β3Gal-T4 and β3Gal-T5 in the tissues of all patients.
As shown in Example 1, specificities of the monoclonal antibody and the polyclonal antisera obtained by immunizing β3Gal-T5 were examined using other β1,3-galactosyltransferase. As a result, it was revealed that both β3Gal-T4 and β3Gal-T5 can be detected by an ELISA system by means of the use of the monoclonal antibody and the polyclonal antisera.
It is considered that ovarian cancer includes ovarian cancer cells secreting β3Gal-T4 and β3Gal-T5 to blood, ovarian cancer cells secreting β3Gal-T4 only to blood, and ovarian cancer cells secreting β3Gal-T5 only to blood. Therefore, it is considered that β1,3-galactosyltransferase, which is present in the sera of ovarian cancer patients and measured by the above ELISA system, may be β3Gal-T4 and/or β3Gal-T5.
Further, it is considered that endometrial cancer includes endometrial cancer cells secreting β3Gal-T4 and β3Gal-T5 to blood, endometrial cancer cells secreting β3Gal-T4 only to blood, and endometrial cancer cells secreting β3Gal-T5 only to blood. Therefore, it is considered that β1,3-galactosyltransferase, which is present in the sera of ovarian cancer patients and measured by the above ELISA system, may be β3Gal-T4 and/or β3Gal-T5.

In the present invention, the method for analyzingβ3Gal-T4 and/or β3Gal-T5 is not particularly limited, so long as it can determine an amount of β3Gal-T4 and/or β3Gal-T5 quantitatively or semiquantitatively, or determine the presence or absence of β3Gal-T4 and/or β3Gal-T5. There may be mentioned, for example, an immunoassay using antibody or fragment thereof against β3Gal-T4 and β3Gal-T5
, such as enzyme immunoassay, latex agglutination assay, chemiluminescent immunoassay, fluorescence antibody technique, radioimmunoassay, immunoprecipitation technique, immunohistochemical assay, or Western blot analysis; a biochemical assay such as enzymatic analysis; and a molecular biological assay in which mRNA is measured.

When the immunoassay is used as the method for analyzing β3Gal-T4 and β3Gal-T5, the monoclonal or polyclonal antibody which binds to β3Gal-T4 and β3Gal-T5 may be used. Alternatively, a combination of a monoclonal or polyclonal antibody which specifically binds to β3Gal-T4, and a monoclonal or polyclonal antibody which specifically binds to β3Gal-T5, may be used.
When the immunoassay is used as the method for analyzing β3Gal-T4, the monoclonal or polyclonal antibody which binds to β3Gal-T4 may be used. In addition, the monoclonal or polyclonal antibody which binds to β3Gal-T4 and β3Gal-T5 may be used.
When the immunoassay is used as the method for analyzing β3Gal-T5, the monoclonal or polyclonal antibody which binds to β3Gal-T5 may be used. In addition, the monoclonal or polyclonal antibody which binds to β3Gal-T4 and β3Gal-T5 may be used.

The monoclonal antibody or polyclonal antibody may be produced according to known methods, except that β3Gal-T4 or β3Gal-T5 is used as an antigen for immunization. For example, the monoclonal antibody is prepared according to a cell fusion method of Kohler and Milstein (Kohler, G. and Milstein, C., Nature, 256, 495-497, 1975). In a preparation of polyclonal antibody, for example, β3Gal-T4 or β3Gal-T5 is conjugated to BSA or KLH. The conjugated β3Gal-T4 or β3Gal-T5, or unconjugated β3Gal-T4 or β3Gal-T5 is emulsified in an appropriate adjuvant (for example, Freund's complete adjuvant), and rabbits are routinely immunized intradermally by using them. If an antibody titer in blood is increased, blood is collected and can be used as an antiserum or an antibody purified by the known methods. In addition, a mouse or rabbit can be immunized with both β3Gal-T4 and β3Gal-5, to obtain a monoclonal antibody or polyclonal antibody which can bind to both β3Gal-T4 and β3Gal-T5.
When the biochemical assay is used as the method for analyzing β3Gal-T4 or β3Gal-T5, for example, the enzymatic activities of β3Gal-T4 or β3Gal-T5 can be measured in accordance with methods of Miyazaki et al. [Miyazaki et al., J. Biol. Chem., (1997) 272, 24794-24799] or Seko et al. [Seko et al., Cancer Res., (1996) 56, 3468-3473], whereby an amount of β3Gal-T4 or β3Gal-T5 or the presence or absence of β3Gal-T4 or β3Gal-T5 can be analyzed.

As an immunizing antigen for obtaining a monoclonal or polyclonal antibody against β3Gal-T5, a protein consisting of amino acid sequence of SEQ ID NO: 33, or a peptide consisting of a part thereof can be used. Specifically, an antigen purified from biological samples, a recombinant antigen prepared by a genetic engineering, or a partial peptide chemically synthesized can be used. In the case of the recombinant antigen expressed by using E. coli, yeast or the like, a fusion protein fused with other protein such as SOD or TrpE can be prepared, in order to express easily. In addition, a fusion protein fused with His-Tag can be prepared in order to purify easily. In particular, β3Gal-T5 wherein cytoplasmic domain and transmembrane domain are removed, is preferably expressed. For example, a polypeptide consisting of the 27th to 310th amino acids in the amino acid sequence of SEQ ID NO: 33, is preferably expressed as the recombinant protein. The monoclonal antibody and polyclonal antibody may be obtained by immunizing the recombinant polypeptide consisting of the 27th to 310th amino acids in the amino acid sequence of SEQ ID NO: 33 as an immunizing antigen.

An antibody binding to β3Gal-T4 and β3Gal-T5, which may be used in the method for detecting ovarian cancer or endometrial cancer of the present invention, is not limited, so long as it can at least bind β3Gal-T4 and β3Gal-T5. As the above antibody, there may be mentioned an antibody which also binds β1,3-galactosyltransferase other than β3Gal-T4 and β3Gal-T5, but preferably, an antibody which specifically binds β3Gal-T4 and β3Gal-T5.
An antibody binding to β3Gal-T4, which may be used in the method for detecting ovarian cancer or endometrial cancer of the present invention, is not limited, so long as it can at least bind β3Gal-T4. As the above antibody, there may be mentioned an antibody which also binds β1,3-galactosyltransferase other than β3Gal-T4, but preferably, an antibody which specifically binds β3Gal-T4.
An antibody binding to β3Gal-T5, which may be used in the method for detecting ovarian cancer or endometrial cancer of the present invention, is not limited, so long as it can at least bind β3Gal-T5. As the above antibody, there may be mentioned an antibody which also binds β1,3-galactosyltransferase other than β3Gal-T5, but preferably, an antibody which specifically binds β3Gal-T5.

As an immunizing antigen for obtaining a monoclonal or polyclonal antibody against β3Gal-T4, a protein consisting of amino acid sequence of SEQ ID NO: 34, or a peptide consisting of a part thereof can be used.

### [1-4] Analysis of GlcNAc6ST-2

In the present invention, ovarian cancer or endometrial cancer are N-acetylglucosaminyl 6-O-sulfotransferase family, and transfers a sulfate group from an adenosine-3'-phosphate-5'phosphosulfate to a GlcNAc residue of various glycoprotein. In normal human, GlcNAc6ST-2 is expressed specifically in high endothelial cells, and is involved with a synthesis of L-selectin ligand.
The present inventors measured enzymatic activity of GlcNAc6ST-2 in tissues of five ovarian cancer patients, and could detect the enzymatic activity of GlcNAc6ST-2 in the tissues of all patients.

In the present invention, the method for analyzing GlcNAc6ST-2 is not particularly limited, so long as it can determine an amount of GlcNAc6ST-2 quantitatively or semiquantitatively, or determine the presence or absence of GlcNAc6ST-2. There may be mentioned for example, an immunoassay using antibody or fragment thereof against GlncNAc6ST-2
, such as enzyme immunoassay, latex agglutination assay, chemiluminescent immunoassay, fluorescence antibody technique, radioimmunoassay, immunoprecipitation technique, immunohistochemical assay, or Western blot analysis; a biochemical assay such as enzymatic analysis; and a molecular biological assay in which mRNA is measured.

When the immunoassay is used as the method for analyzing GlcNAc6ST-2, the monoclonal or polyclonal antibody which binds to pGlcNAc6ST-2, may be used. The monoclonal antibody or polyclonal antibody may be produced according to known methods, except that GlcNAc6ST-2 is used as an antigen for immunization. For example, the monoclonal antibody is prepared according to the cell fusion method of Kohler and Milstein (Kohler, G. and Milstein, C., Nature, 256, 495-497, 1975). In a preparation of polyclonal antibody, for example, GlcNAc6ST-2 is conjugated to BSA or KLH. The conjugated GlcNAc6ST-2, or unconjugated GlcNAc6ST-2 is emulsified in an appropriate adjuvant (for example, Freund's complete adjuvant) and, rabbits are routinely immunized intradermally by using the same. If an antibody titer in blood is increased, blood is collected and can be used as an antisera or an antibody purified by known methods.
When the biochemical assay is used as the method for analyzing GlcNAc6ST-2, for example, the enzymatic activity of GlcNAc6ST-2 can be measured in accordance with method of Seko et al. [Seko et al., Glycobiology (2002) 12, 379-388], whereby an amount of GlcNAc6ST-2 or the presence or absence of GlcNAc6ST-2 can be analyzed.

As an immunizing antigen for obtaining a monoclonal or polyclonal antibody against GlcNAc6ST-2, a protein consisting of amino acid sequence of SEQ ID NO: 35, or a polypeptide consisting of a part thereof can be used. Specifically, an antigen purified from a biological sample, a recombinant antigen prepared by genetic engineering, or a partial peptide chemically synthesized can be used. In the case of the recombinant antigen expressed by using E. coli, yeast or the like, a fusion protein fused with other protein, such as SOD or TrpE can be prepared, in order to express easily. In addition, a fusion protein fused with His-Tag can be prepared, in order to purify easily. In particular, GlcNAc6ST-2, wherein cytoplasmic domain and transmembrane domain are removed, is preferably expressed. For example, a polypeptide consisting of the 28th to 386th amino acids in the amino acid sequence of SEQ ID NO: 35, is preferably expressed as a recombinant protein.

An antibody binding to GlcNAc6ST-2, which may be used in the method for detecting ovarian cancer or endometrial cancer of the present invention, is not limited, so long as it can at least bind GlcNAc6ST-2. As the above antibody, there may be mentioned an antibody which also binds GlcNAc6ST other than GlcNAc6ST-2, but preferably, an antibody which specifically binds GlcNAc6ST-2.

### [1-5] Immunological analysis

When the enzyme immunoassay, for example a sandwich immunoassay, is used as the immunoassay, the sandwich immunoassay can be carried out according to the following procedure. The procedure of the sandwich immunoassay for GlcNAc6ST-2 is described in an illustration.
An antibody or an antibody fragment (a captured antibody or first antibody) binding to GlcNAc6ST-2 is immobilized to an appropriate insoluble carrier, such as a microtiter plate or a micro bead. Then the insoluble carrier is coated with an appropriate blocking agent, such as bovine serum albumin (BSA) or gelatin, to prevent a nonspecific binding of a sample to the insoluble carrier. Thereafter, the sample which may contain GlcNAc6ST-2, and first reaction buffer are added to the microtiter plate or the micro bead. Then GlcNAc6ST-2 in the sample is brought into contact with the captured antibody, to perform a reaction (First reaction process). Then, unbinding antigen (GlcNAc6ST-2) or other substance in the sample is washed with an appropriate wash buffer, for example, phosphate buffer including detergent. Then, labeled antibody in which an antibody binding to the captured GlcNAc6ST-2 is conjugated to an enzyme such as horseradish peroxidase (HRP), is added to the whole, so as to bind the labeled antibody to the captured antigen (second reaction process), and whereby immune complex (i.e. the captured antibody-GlcNAc6ST-2-labeled antibody complex) is formed on the insoluble carrier such as the microtiter plate. An unbinding labeled antibody is washed with an appropriate wash buffer, and then a colorimetric substrate or a luminescent substrate for the enzyme of the labeled antibody is added. A detectable signal may be developed by a reaction in the enzyme and the substrate.
Alternatively, a labeled antibody, which may bind to the second antibody, can be used instead of the labeled second antibody, in order to detect the signal.
As the enzyme for labeling the antibody, there may be mentioned a horseradish peroxidase (HRP), alkaline phosphatase, β-galactosidase, luciferase and the like. In addition, a luminescent material such as acridinium derivatives, a fluorescent material such as europium, or radioactive material such as I¹²⁵ can be used as a labeling material other than the enzyme. Further, a substance and a material for inducing luminescence are appropriately selected in accordance with the labeling material. The labeled antibody used in the present invention contains an antibody conjugated with a hapten, low molecular weight peptide, and a lectin which can be used for detecting the signal of antigen-antibody complex reaction.
In a sandwich immunoassay of β3Gal-T4 and/or β3Gal-T5, the sandwich immunoassay system may be constructed by replacing the antibody binding to GlcNAc6ST-2 with an antibody binding to β3Gal-T4 and/or β3Gal-T5 in the above sandwich immunoassay system of GlcNAc6ST-2.

If either the first antibody or the second antibody may specifically bind the protein to be analyzed, the other antibody, which may bind to a protein other than the protein to be analyzed or may exhibit cross-reactivity to the other protein, may be used in the sandwich ELISA. In the analysis of β3Gal-T4 and β3Gal-T5, for example, if either the first antibody or the second antibody may specifically bind β3Gal-T4 and β3Gal-T5, β3Gal-T4 and β3Gal-T5 can be analyzed specifically.
Further, in the analysis of GlcNAc6ST-2, if either the first antibody or the second antibody may specifically bind GlcNAc6ST-2, GlcNAc6ST-2 can be analyzed specifically. Furthermore, in the analysis of β3Gal-T4, if either the first antibody or the second antibody may specifically bind β3Gal-T4, β3Gal-T4 can be analyzed specifically, In addition, in the analysis of β3Gal-T5, if either the first antibody or the second antibody may specifically bind β3Gal-T5, β3Gal-T5 can be analyzed specifically.

The first antibody and the second antibody used in the sandwich immunoassay contains, but is not particularly limited to, a polyclonal antibody, monoclonal antibody, recombinant antibody, fragment thereof or the like. As the fragment of the antibody, there may be mentioned F(ab')₂, Fab', Fab, Fv, or the like. The fragment of the antibody may be obtained by conventional methods, for example, by digesting the antibody using a protease (such as pepsin, papain, or the like) and purifying the resulting fragments by standard polypeptide isolation and purification methods.

In the method for detecting ovarian cancer or endometrial cancer of the present invention, as a sample to be tested, which is used for analyzing β3Gal-T4 and/or β3Gal-T5, or GlcNAc6ST-2 as defined in claims 2, 3, 10 and 11 to be blood, serum or plasma. In other embodiments of the invention it may be urine, blood, serum, plasma, spinal, fluid, saliva, cell, tissue, organ, or preparation thereof (for example, biopsy specimen, particularly biopsy specimen of ovary) or the like. The sample to be tested is preferably blood, serum, plasma, or biopsy specimen of ovary, more preferably blood, serum, or plasma (hereinafter referred to as a blood or the like), but following the definitions of the claims. β3Gal-T4 and/or β3Gal-T5, GlcNAc6ST-2 are not contained in the blood, serum, and plasma of normal humans. On the other hand, β3Gal-T4 and/or β3Gal-T5, or GlcNAc6ST-2 are released to the blood of ovarian cancer patients or endometrial cancer patients at an early stage of the cancer. Therefore, the blood or the like is appropriate as the sample to be tested for detecting ovarian cancer or endometrial cancer.

In the present invention, ovarian cancer or endometrial cancer can be detected by determining the presence or absence of β3Gal-T4 and/or β3Gal-T5, or GlcNAc6ST-2, or determining an amount of β3Gal-T4 and/or β3Gal-T5, or GlcNAc6ST-2 quantitatively or semiquantitatively. For example, when the biopsy specimen of the ovary or the uterus is used as the sample to be tested, the expression of β3Gal-T4 and/or β3Gal-T5, or GlcNAc6ST-2 can be determined by the immunohistochemical assay using the monoclonal or the polyclonal antibody, whereby it can be determined whether or not the sample is one of ovarian cancer, or the sample is one of endometrial cancer.
Further, when the blood or the like is used as the sample to be tested, blood is collected from a patient possibly suffering from ovarian cancer or endometrial cancer, then an amount of GlcNAc6ST-2 is measured using the collected blood or serum or plasma thereof. Then it can be determined whether or not the patient has ovarian cancer, or the patient has endometrial cancer by comparing the amount of GlcNAc6ST-2 of the patient with that of a normal human. More particularly, if the amount of GlcNAc6ST-2 in the patient is significantly high in comparison to that of a normal human, the patient can be diagnosed with ovarian cancer. In addition, as to β3Gal-T4 and β3Gal-T5, β3Gal-T4, or β3Gal-T5, it is possible to diagnose with ovarian cancer by comparing β3Gal-T4 and β3Gal-T5, β3Gal-T4, or β3Gal-T5 in the patients with that of a normal human.
For example, in the case of after-mentioned sandwich ELISA, the average of an amount of β3Gal-T4 and/or β3Gal-T5, or GlcNAc6ST-2 in a normal human is calculated, then the standard deviation (SD) thereof is calculated. In the present invention, the cut-off point for detecting the cancer patient is not limited, so long as each cancer can be detected by using the cut-off point. It is possible to define an average + SD, average + 2SD, or average + 3SD as cut-off point, and further, it is also possible that a sample having a value higher than average is diagnosed as positive one.

In the detection of ovarian cancer or endometrial cancer of the present invention, most preferably the ovarian cancer or endometrial cancer is specifically detected. However, if an early diagnosis of ovarian cancer by a blood test in a health examination is needed, preferably ovarian cancer patients or endometrial cancer patients can be detected preliminary. That is to say, it is preferable that the tumor marker can detect cancer patients as much as possible, among the ovarian cancer patients or endometrial cancer patients. According to the detecting method of the present invention, as shown in Examples below, the ovarian cancer patients (not including a patient supervening on other cancer) confirmed by the operation, or the confirmed endometrial cancer patients, can be detected at high rates. Therefore, the detecting method of the present invention is useful as a cancer marker.

### [2] Kit for detecting ovarian cancer or endometrial cancer

The kit for detecting ovarian cancer or endometrial cancer of the present invention may contain the antibody or fragment thereof specifically binding to β3Gal-T4 and β3Gal-T5, and the antibody or fragment thereof specifically binding to GlcNAc6ST-2.
As the antibody, a monoclonal antibody or a polyclonal antibody can be contained therein. The fragment of antibody is not particularly limited, so long as it can have specific binding ability to β3Gal-T4 and β3Gal-T5, or specific binding ability to GlcNAc6ST-2. For example, antibody fragment Fab, Fab', F(ab')₂, or Fv can be contained therein.
The detecting kit of the present invention may contain the antibody or fragment thereof specifically binding to β3Gal-T4 and β3Gal-T5, and the antibody specifically binding to GlcNAc6ST-2 or a fragment thereof.

The detecting kit of the present invention contains a desired form of antibody specifically binding to β3Gal-T4 and β3Gal-T5, or a fragment thereof, or desired form of antibody specifically binding to GlcNAc6ST-2, or a fragment thereof in accordance with the immunoassay used in the detecting kit.
For example, in a case of an immunoassay using the labeled antibody, such as enzyme immunoassay, chemiluminescent immunoassay, fluorescence antibody technique, or radioimmunoassay, the labeled antibody or antibody fragment conjugated by a labeling material can be contained therein. Specifically, the labeling material can include peroxidase, alkaline phosphatase, β-D-galactosidase, or glucose oxidase as an enzyme; fluorescein isothiocyanate, rare-earth metal chelate as a fluorescent material; ³H, ¹⁴C, or ¹²⁵I as a radioactive isotope; or biotin, avidin, or a chemiluminescent substance. In addition, in the case of an enzyme or a chemiluminescent substance, since they cannot develop a measurable signal by themselves, a substance corresponding to each enzyme or chemiluminescent substance is preferably contained therein.

### EXAMPLES

The present invention now will be further illustrated by, but is by no means limited to, the following Examples and Comparative Examples.

### Example 1: Preparation of anti β3Gal-T5 antibody

### (A) Preparation of soluble form of β3Gal-T5 (Preparation of antigen for immunization)

### (A-1) Construction of β3Gal-T5 expression vector

A cDNA fragment encoding a peptide lacking cytoplasmic domain and transmembrane domain was amplified by PCR. The PCR was carried out by using a human colon cDNA library and the following Forward primer for b3Gal-T5 and Reverse primer for b3Gal-T5. The obtained cDNA fragment encodes a peptide consisting of the 27th to 310th amino acids in the amino acid sequence of β3Gal-T5.
Forward primer for b3Gal-T5: 5'-tttgtcgacAGTCTAAATCCTTTCAAAG-3' (SEQ ID NO: 1)
Reverse primer for b3Gal-T5: 5'-tttaagcttCAGACAGGCGGACAATC-3' (SEQ ID NO: 2)
In the above sequences, the sequences in lowercase letters contain appropriate restriction sites to be fused to an expression vector. The obtained cDNAs were inserted into a cloning site of pQE9 vector (QIAGEN GmbH, Hilden, Germany), then *E. coli* M15 cells were transformed with the resulting vector. The nucleotide sequences of the obtained plasmids were determined with a Prism 310 Genetic Analyzer (Applied Biosystems, Foster City, CA).

### (A-2) Expression and purification of β3Gal-T5

Preparation of recombinant β3Gal-T5 was as follows. The overnight culture of transformed *E. coli* M15 cells were diluted by 1/100, added to 15L of LB broth containing 100µg/mL ampicillin and 25µg/mL kanamycin, and grew at 30 °C for 3 hr with vigorous stirring. Isopropyl β-D-1-thiogalactopyranoside (hereafter, referred to as IPTG) having a final concentration of 0.1mM was added to the culture and the expression of β3Gal-T5 was induced at 18 °C for 16 hr. The *E. coli* cells were collected with centrifugation and sonicated in Buffer A [200mL of 6M guanidine-HCl, 20mM potassium phosphate buffer (pH 8.0), 10mM 2-mercaptoethanol, and 20mM imidazole]. The obtained homogenate was allowed to stand at 25 °C for 16 hr. After centrifugation at 5,000 g for 20 min, the supernatant was applied to 3mL of Ni-NTA agarose (QIAGEN GmbH) and allowed to stand for 30 min with occasional mixing. The resin was packed into a plastic column and washed with 60mL of Buffer A. Thereafter, the recombinant protein was eluted with Buffer A containing 0.25M imidazole. The eluate was dialyzed against 8M urea-PBS, and concentrated with a Microcon YM-10 (Millipore Corp., Bedford, MA). Finally, 2.5mg of the recombinant protein was obtained. The obtained protein was referred as denatured β3Gal-T5 (*E. coli*).

### (B) Preparation of soluble form of non-denatured β3Gal-T5 (Preparation of antigen for screening)

Using the same transformed *E. coli* M15 as prepared in Example 1, non-denatured β3Gal-T5 was prepared. After induction with 0.1mM IPTG in 300mL *E. coli* cell culture, the cells were collected and sonicated in 25mL of 20mM potassium phosphate buffer (pH 8.0)(containing 0.3M NaCl and 10mM imidazole). To the obtained homogenate, 2.5mL of 5% Triton X-100 (v/v) was added and the mixture was allowed to stand on ice for 30 min. After centrifugation, 0.3mL of Ni-NTA agarose was added to the supernatant, and the mixture was allowed to stand for 30 min with occasional mixing. The resin was packed into a plastic column and washed with 5mL of Buffer B (20mM potassium phosphate buffer (pH 8.0), 0.3M NaCl, and 20mM imidazole) containing 0.5% Triton X-100, and further washed with another 5mL of Buffer B. The recombinant protein was eluted with Buffer B containing 0.25M imidazole. The eluate was washed with PBS using a Microcon YM-10 (Millipore Corp., Bedford, MA) then concentrated. Among 0.36mg of finally obtained proteins, recombinant β3Gal-T5 was approximately 10 % yield, from estimation by SDS-PAGE analysis. It should be noted that this fraction contained substantial β3Gal-T5 activity. The obtained protein was referred to as denatured β3Gal-T5 (E. coli).

### (C) Preparation of monoclonal and polyclonal antibodies

The monoclonal and the polyclonal antibodies against β3Gal-T5 were commercially prepared by Medical and Biological Laboratories Co., Ltd. (Nagoya, Japan). The monoclonal antibodies were prepared as follows. The above denatured β3Gal-T5 *(E. coli)* was injected three times into the footpads of four mice at three day intervals. In accordance with the conventional method, mouse spleen cells were fused with mouse myeloma cells, then the screening of hybridomas for producing antibodies were carried out by using the non-denatured β3Gal-T5 (*E. coli*) as the antigen. A hybridoma for producing monoclonal antibody which binds to the non-denatured β3Gal-T5 was cloned. In accordance with the conventional method, the hybridoma was intraperitoneally-inoculated to five mice, to obtain 25mL of ascites. The ascitic fluid was purified with Sephacryl S-300gel filtration (1.4 × 68cm; equilibrated and eluted with TBS: GE Healthcare) to obtain 21mg of β3Gal-T5 monoclonal antibody.
In the case of polyclonal antibody, the non-denatured β3Gal-T5 (*E. coli*) was injected six times into a rabbit at one week intervals to obtain β3Gal-T5 polyclonal antisera.

### (D) Identification of specificities in monoclonal and polyclonal antibodies

To identify the specificities in the resulting monoclonal and the polyclonal antibodies with respect to β3Gal-T5, reactivity with respect to each of β3GalT-1, β3GalT-2, β3Gal-T4, β3GalT-6, and β3GalNAc-T1 was examined, respectively. First, the recombinant protein for each of β3GalT-1, β3GalT-2, β3Gal-T4, β3GalT-6, and β3GalNAc-T1 was prepared. The step (A-1) was repeated to construct an expression vector, except that the following primers were used as a primer for β3GalT-1. On the other hand, for β3GalT-2, β3Gal-T4, β3GalT-6, and β3GalNAc-T1, the step (A-1) was repeated to construct an expression vector, except that a Super Script human brain cDNA library was used as cDNA library instead of a human colon cDNA library, and the following primers were used as primer.
Forward primer for β3Gal-T1: 5'-tttggatccAGTATAACTCGCCCTACTT-3' (SEQ ID NO: 3)
Reverse primer for β3Gal-T1: 5'-tttaagcttCCTAACATCTGAGATG-3' (SEQ ID NO: 4)
Forward primer for β3Gal-T2: 5'-tttggatccCTGCCAGGCAGAGCTGGA-3' (SEQ ID NO: 5)
Reverse primer for β3Gal-T2: 5'-tttaagcttAATGTAGTTTACGGTGG-3' (SEQ ID NO: 6)
Forward primer for β3GalNAc-T1: 5'-tttggatccAGCCTTCCCCACTACAATG-3' (SEQ ID NO: 7)
Reverse primer for β3GalNAc-T1: 5'-tttaagcttAATAATGGCATGTGGTGT-3' (SEQ ID NO: 8)
Forward primer for β3Gal-T4: 5'-tttggatccTTGGGGGAGGAGCTGCTG-3' (SEQ ID NO: 9)
Reverse primer for β3Gal-T4: 5'-tttgtcgacTCAGCTCTGAAGCCAGGC-3' (SEQ ID NO: 10)
Forward primer for β3Gal-T6: 5'-tttggatccGACCCCAGGGCGATGTCG-3' (SEQ ID NO: 11)
Reverse primer for β3Gal-T6: 5'-tttaagcttGGCTCAGGGGATGCCCT-3' (SEQ ID NO: 12)

Each recombinant protein was prepared by carrying out step (A-2) with few modifications. Concretely, one hundredth part of each overnight culture of the transformed M15 cells was added to 15mL of LB broth containing the antibiotics, and cultured at 30 °C for 3 hr. IPTG having a final concentration of 0.1mM was added to the cultures and the expression was induced at 18 °C for 16 hr. The *E. coli* cells were collected and sonicated in 10mL of Buffer A. The homogenates were allowed to stand at 25 °C for 16 hr. After centrifugation, the supernatants were added to 0.5mL of Ni-NTA agarose (QIAGEN GmbH) and were allowed to stand for 30 min. The resins were washed with 10mL of Buffer A, and the recombinant proteins were eluted with Buffer A containing 0.25M imidazole. As a result, the yields for β3GalT-1, β3GalT-2, β3Gal-T4, β3GalT-6, and β3GalNAc-T1 were 0.03mg, 0.03mg, 0.06mg, 0.70mg, and 1.8mg, respectively.

Then the reactivities were identified by using Western blotting analysis. 300ng of each obtained protein was loaded with polyacrylamide gel, and transferred to a nitrocellulose membrane. After blocking, the membrane was incubated with anti β3Gal-T5 monoclonal antibody (1µg/mL). The membrane was washed, and further incubated with HRP labeled anti mouse IgG/IgM rabbit antibody (0.3µg/mL: Jackson ImmunoReseach). The chemiluminescence detection was carried out using ECL Western blot detection reagent (GE Healthcare).
For the reactivities on in polyclonal antisera, 50ng of each protein was loaded, and a nitrocellulose membrane was incubated with the polyclonal antisera diluted by a factor of 1000. After washing, the membrane was incubated with a HRP labeled anti rabbit Ig antibody. Then Chemiluminescence detection was carried out as is in the case of the monoclonal antibody.
As shown in Fig. 1, the monoclonal antibody showed an intense reactivity to β3Gal-T5. Further, it also showed reactivities for β3GalT-1 and β3Gal-T4. On the other hand, the polyclonal antibody showed an intense reactivity for β3Gal-T5, as well as reactivity for β3Gal-T4.

### Example 2: Expression of mRNAs of β3Gal-T4 and β3Gal-T5 in ovarian cancer cells

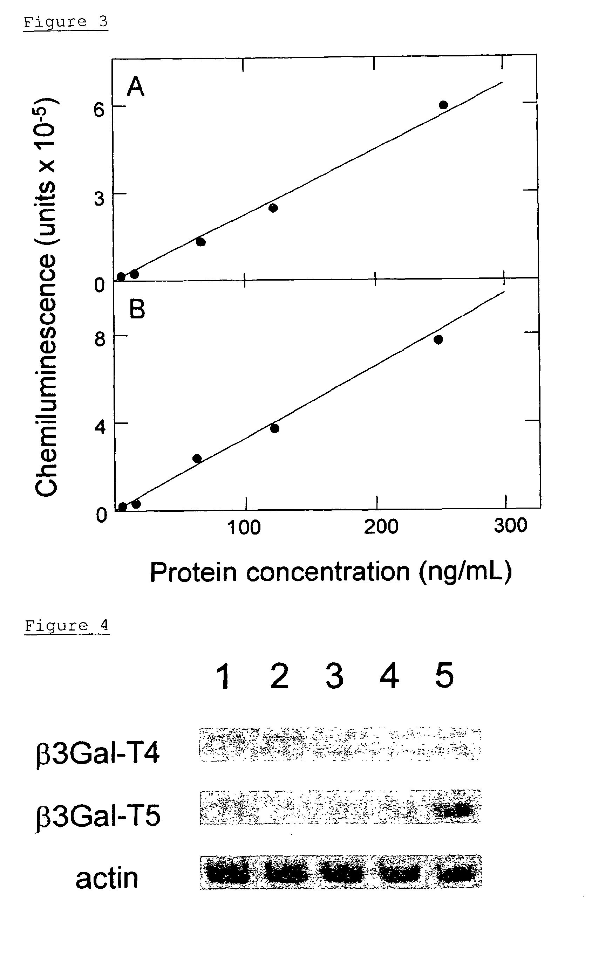

The Expression of mRNAs of β3Gal-T4 and β3Gal-T5 in ovarian cancer cells was investigated by RT-PCR using five different types of ovarian cancer cells. ES-2 cells (ovarian clear cell carcinoma) were purchased from ATCC, and MCAS cells (ovarian mucinous cystadenocarcinoma), RMG-I cells (ovarian mesonephroid adenocarcinoma), RMG-II cells (ovarian mesonephroid adenocarcinoma), and RMUG-L cells (ovarian mucinous cystadenocarcinoma) were purchased from Human Science Research Resources Bank (Osaka, Japan).
The harvested cells were lysed using ISOGEN (Nippon Gene, Japan) to isolate total RNAs. The cDNAs were synthesized from the obtained RNAs by using oligo(dT) primers and SuperScript III (Invitrogen). The amounts of cDNAs were normalized by those of β-actin (Seko *et al.,* 2002).
The following primers were added to the cDNAs, and the incubation was carried out at 95°C for 2 min. Afterward, the expression of β3Gal-T4 and β3Gal-T5 was examined by PCR. PCR was performed using GoTaq DNA polymerase (Promega) in a volume of 20µL for 29cycles of 95°C for 30 seconds, 62°C for 1 minute, and 72°C for 2 minutes.
Forward primer for PCR of β3Gal-T4: 5'-ATTCTTGCTGGGAGAGCCGAAC-3' (SEQ ID NO: 13)
Reverse primer for PCR of β3Gal-T4: 5'-AAGTACAGAAGAGGCACTTCCTC-3' (SEQ ID NO: 14)
Forward primer for PCR of β3Gal-T5: 5'-GGAAACGAAAGAGGTGGACCAG-3' (SEQ ID NO: 15)
Reverse primer for PCR of β3Gal-T5: 5'-GAGCTCCTCCAATCTGATGTTCA-3' (SEQ ID NO: 16)
As shown in Fig. 4, mRNA of β3Gal-T4 was expressed in each of ES-2 cells, MCAS cells, RMG-I cells, and RMUG-L cells. On the other hand, mRNA of β3Gal-T5 was expressed in each of ES-2 cells, RMG-I cells, RMG-II cells, and RMUG-L cells.

mRNA of β3Gal-T4 and mRNA of β3Gal-T5 were expressed in four ovarian cancer cell strains among the five ovarian cancer cell strains. Further, in the five ovarian cancer cell strains, either one of mRNA of β3Gal-T4 or mRNA of β3Gal-T5 was expressed.

### Example 3: Preparation of GlcNAc6ST-2 antibody

### (A) Preparation of soluble form of GlcNAc6ST-2 (Preparation of antigen for immunization)

### (A-1) Construction of GlcNAc6ST-2 expression vector

A cDNA fragment encoding a peptide lacking cytoplasma and transmembrane domains was amplified by PCR. The PCR was carried out using a full-length cDNA prepared previously (Glycobiology, 2002, 12, p.379-388) and the following Forward primer for GlcNAc6ST2 and Reverse primer for GlcNAc6ST2.
The obtained cDNA fragment encodes a peptide consisting of the 28th to 386th amino acids in the amino acid sequence of of GlcNAc6ST-2.
Forward primer for GlcNAc6ST2: 5'-tttgtcgacAGCCACAACATCAGCT-3' (SEQ ID NO: 17)
Reverse primer for GlcNAc6ST2: 5'-tttaagcttAGTGGATTTGCTCAG-3' (SEQ ID NO: 18)
In the above sequences, the sequences in lowercase letters contain appropriate restriction sites to be fused to an expression vector. The obtained cDNAs were inserted into a cloning site of pQE9 vector (QIAGEN GmbH, Hilden, Germany), and then, *E. coli* M15 cells were transformed with the resulting vector. The nucleotide sequences of the obtained plasmids were determined with a Prism 310 Genetic Analyzer (Applied Biosystems, Foster City, CA).

### (A-2) Expression and purification of GlcNAc6ST-2

The step (A-2) in Example 1 was repeated to obtain 3.5mg of GlcNAc6ST-2, except that the culture for expression induction by IPTG was carried out at 1.25L using a GlcNAc6ST-2 expression vector instead of a β3Gal-T5 expression vector. The obtained proteins were referred as *E. coli* GlcNAc6ST-2.

### (B) Preparation of soluble form of GlcNAc6ST-2 in Pichia pastoris (Preparation of antigen for screening)

GlcNAc6ST-2 was prepared by using yeasts for screening monoclonal antibodies. A cDNA fragment encoding a peptide laking cytoplasma and transmembrane domains was amplified by PCR. The PCR was carried out by using a full-length cDNA prepared previously (Glycobiology, 12, p.379-388, 2002) and the following Forward primers for GlcNAc6ST2-pichia and Reverse primer for GlcNAc6ST2-pichia.
Forward primer for GlcNAc6ST2-pichia: 5'-tttcctaggTACAGCCACAACATCAG-3' (SEQ ID NO: 19)
Reverse primer for GlcNAc6ST2-pichia: 5'-tttgcggccgcTTAGTGGATTTGCTCAGG-3' (SEQ ID NO: 20)
The obtained cDNA was inserted into cloning sites appropriate for pPIC9-His vector and the nucleotide sequence was determined with a Prism 310 Genetic Analyzer. Production and purification of the recombinant GlcNAc6ST-2 was carried out in accordance with the known method (Glycobiology 15 p. 943-951, 2005). From 500mL of a culture in buffered methanol-complex medium, 300µg of the recombinant protein was obtained. It should be noted that this fraction contained substantial GlcNAc6ST-2 activity. The obtained protein was referred to as yeast GlcNAc6ST-2.

### (C) Preparation of monoclonal and polyclonal antibodies

Step (C) in Example 1 was repeated to obtain a strain of hybridoma for producing monoclonal antibody and to obtain polyclonal antisera, except that *E. coli* GlcNAc6ST-2 cells were used as immunogen and that yeast GlcNAc6ST-2 was used as screening antigen for the monoclonal antibody. The hybridoma was intraperitoneally-inoculated into five mice, to obtain 13mL of ascites. The obtained ascetic fluid was purified with protein A-Sepharose affinity chromatography (0.9 × 7.9cm; equilibrated with PBS: GE Healthcare) to obtain 12.6mg of antibody.

### (D) Identification of specificities in monoclonal and polyclonal antibodies

To identify the specificities in the resulting monoclonal and polyclonal antibodies with respect to GlcNAc6ST-2, the reactivity with respect to each of GlcNAcc6STl, GlcNAcc6ST3, GlcNAcc6ST4, GlcNAcc6ST5, Gal6ST, and Ch6ST1 was examined, respectively. First, the recombinant protein for each of GlcNAcc6STl, GlcNAcc6ST3, GlcNAcc6ST4, GlcNAcc6ST5, Gal6ST, and Ch6ST1 was prepared. Then, step (A-1) in Example 1 was repeated to construct an expression vector, except that the following primers were used.
Forward primer for GlcNAc6ST1: 5'-tttgtcgacTACAAGTGGCACAAG-3' (SEQ ID NO: 21)
Reverse primer for GlcNAc6ST1: 5'-tttgtcgacCCCTTTTAGAGACGG-3' (SEQ ID NO: 22)
Forward primer for GlcNAc6ST3: 5'-tttgtcgacTCCCGGCCAGGGCCCTC-3' (SEQ ID NO: 23)
Reverse primer for GlcNAc6ST3: 5'-tttaagcttCAGTCAGGCGATGCCCA-3' (SEQ ID NO: 24)
Forward primer for GlcNAc6ST4: 5'-tttgtcgacGGCGGCCGCGACGG-3' (SEQ ID NO: 25)
Reverse primer for GlcNAc6ST4: 5'-tttaagcttGGGAGGCTACGTGGCGC-3' (SEQ ID NO: 26)
Forward primer for GlcNAc6ST5: 5'-tttgtcgacTCCCGGCCAGGGCCCTC-3' (SEQ ID NO: 27)
Reverse primer for GlcNAc6ST5: 5'-tttaagcttGCTACAACTGTGGCCTC-3' (SEQ ID NO: 28)
Forward primer for Gal6ST: 5'-tttgtcgacACCTTCACCGCCAAGTC-3' (SEQ ID NO: 29)
Reverse primer for Gal6ST: 5'-tttaagcttCACGAGAAGGGGCGGAA-3' (SEQ ID NO: 30)
Forward primer for Ch6ST1: 5'-tttgtcgacATATCAAGGGTCTCAGA-3' (SEQ ID NO: 31)
Reverse primer for Ch6ST1: 5'-tttaagcttCTACGTGACCCAGAAGGT-3' (SEQ ID NO: 32)

Step (D) in Example 1 for the expression and purification of β3GalT-1 and so on, was repeated to obtain each recombinant protein, except that 15mL of culture was used for the expression induction by IPTG. The yields for GlcNAcc6ST1, GlcNAcc6ST3, GlcNAcc6ST4, GlcNAcc6ST5, Gal6ST, and Ch6ST1 were 0.20mg, 1.2mg, 0.26mg, 0.83mg, 0.26mg, and 0.13mg, respectively.

**Table 1**

| | | Number of samples | GN6ST2 | *β* 3GaIT5 | GN6ST2 + *β* 3GaIT5 | CA602/125 | CA564 |
|---|---|---|---|---|---|---|---|
| **Disease stages** | **Disease states I to II** | 31 | 74.2 | 87. 1 | 96.8 | 73.1 | 70.8 |
| | **Disease states III to IV** | 29 | 58.6 | 93.1 | 93.1 | 96.3 | 95.5 |
| **Histological types** | **serious adenocarcinoma** | 18 | 44.4 | 88.9 | 88.9 | 93.8 | 92.9 |
| | **mucinous adenocarcinoma** | 10 | 70.0 | 100 | 100 | 71.4 | 85.7 |
| | **endometrioid adenocarcinoma** | 13 | 76.9 | 84.6 | 100 | 77.8 | 83.3 |
| | **clear cell adenocarcinoma** | 16 | 75.0 | 87.5 | 93.8 | 86.7 | 69.2 |
| | **Serous + Endometrioid** | 31 | 58.1 | 87.1 | 93.5 | 88.0 | 90.0 |
| | **Mucinous + Clear cell** | 26 | 73.1 | 92.3 | 96.2 | 81.8 | 75.0 |

| | | | | | | | |
|---|---|---|---|---|---|---|---|
| * Positive rates are given in %. | | | | | | | |

Then the western blotting method was performed. The western blotting step (D) in Example 1 was repeated, except that 200ng of each of the antigens for the electrophoresis was used.
As shown in Fig. 2, the monoclonal antibody showed an intensive reactivity only to GlcNAc6ST-2. On the other hand, the polyclonal antisera showed reactivity to all antigens.

### Example 4: Construction of sandwich ELISA of β3Gal-T5 and GlcNAc6ST-2

Construction of an immunoassay system for β3Gal-T5 and GlcNAc6ST-2 were performed by a sandwich ELISA method using the monoclonal antibody and polyclonal antisera obtained by immunizing the β3Gal-T5, and by using the monoclonal antibody and polyclonal antisera obtained by immunizing the GlcNAc6ST-2.

### (A) β3Gal-T5 detection system

50µL of b3Gal-T5 monoclonal antibody, which had been diluted to a concentration of 1µg/mL with a PBS, was added to 96-well black high-binding plates (Corning Inc., Corning, NY), and was immobilized at 4 °C for 16 hr. The surface of each well was blocked with PBS containing 1 % BSA at 25 °C for 1 hr. The non-denatured β3Gal-T5 (*E. coli*) as reference materials, was diluted to a concentration of 250ng/mL, 125ng/mL, 62.5ng/mL, 16ng/mL, and 4.0ng/mL with a diluent wherein 1% BSA had been added to PBS-0.1% Tween-20 (PBS-T). Then 50µL of the non-denatured β3Gal-T5 (*E. coli*) was added to the wells, and incubated at 25 °C for 2 hr. After washing with PBS-T three times, 50µL of β3Gal-T5 polyclonal antisera diluted by a factor of 1000 was added and incubated at 25°C for 1.5hr. After washing with PBS-T three times, donkey HRP-labeled anti-rabbit Ig antibody (0.3µg/mL: GE Healthcare) diluted with PBS-T was added and incubated at 25 °C for 1 hr. The wells were washed with PBS-T four times, and 100µL of SuperSignal ELISA Pico Chemiluminescent Substrate (PIERCE, Rockford, IL) was added and chemiluminescent quantification was carried out by using a Plate CHAMELEON V (HIDEX Oy, Turku, Finland).

### (B) GlcNAc6ST-2 detection system

Step (A) was repeated to construct a sandwich ELISA of GlcNAc6ST-2, except that the monoclonal antibody having a concentration of 3µL/mL with respect to GlcNAc6ST-2 was immobilized, the polyclonal antisera with respect to GlcNAc6ST-2 was diluted by a factor of 3000, and GlcNAc6ST-2 (*E. coli*) was used as reference material.
As shown in Fig. 3, a good linear standard curve for each of the β3Gal-T5 detection system and the GlcNAc6ST-2 detection system were obtained.

### (C) Improvement in a standard curve for the β3Gal-T5 detection system

Step (A) was repeated to obtain the standard curve for β3Gal-T5, except that non-denatured β3Gal-T5 (*E. coli*) was diluted using PBS-0.1%Tween-20 (PBS-T) wherein normal human serum 10 % was added, instead of using PBS-0.1%Tween-20 (PBS-T). The results are shown in Fig. 5A. At low concentrations of β3Gal-T5, the linearity of the standard curve was improved. Other than the normal human serum, the use of sera of bovine, horse, sheep, goat or the like showed the same effects as obtained in the normal human serum.

### (D) Improvement in a standard curve for GlcNAc6ST-2 detection system

Step (B) was repeated to obtain the standard curve for GlcNAc6ST-2, except that non-denatured GlcNAc6ST-2 (*E. coli*) was diluted using PBS-0.1%Tween-20 (PBS-T) wherein normal human serum 10% was added, instead of using PBS-0.1%Tween-20 (PBS-T). The results are shown in Fig. 5A. At low concentrations of GlcNAc6ST-2, the linearity of the standard curve was improved.

### Example 5: Measurement in sera of ovarian cancer patients using β3Gal-T5 detection system

Sera of 60 cases of ovarian cancer patients and sera of 8 samples of healthy adult women were measured using the β3Gal-T5 detection system. The sera of the ovarian cancer patients to be used in the measurement were obtained from ovarian cancer patients of stages I to IV which were confirmed by abdominal operation. In the 57 cases, it was possible to determine the histological types of ovarian cancer by using histological examination. Any cancer developments other than ovarian cancer were not confirmed in these ovarian cancer patients.
Step (A) in Example 4 was repeated, except that the sera of patients and of healthy persons diluted with PBS-T by a factor of 10 were used instead of non-denatured β3Gal-T5 (*E. coli*). The amount of β3Gal-T5 in the sera was determined by the standard curve (A) in Fig. 3. The value of an average + 2SD in healthy adult women was defined as a cut-off point, and it was determined whether the sera of each patient was positive or negative.
In the examples, the monoclonal antibody and the polyclonal antisera were obtained by immunizing β3Gal-T5 in (D) of Example 1. The reactivities of the monoclonal antibody and the polyclonal antisera with respect to other β1,3-galactosyltransferases were examined. As a result, it is possible to detect both β3Gal-T4 enzyme and β3Gal-T5 enzyme by ELISA using the monoclonal antibody and the polyclonal antisera.

### Example 6: Measurement in sera of ovarian cancer patients by using GlcNAc6ST-2 detection system

Sera of 60 cases of ovarian cancer patients and sera of 8 samples of healthy adult women as tested in Example 5, were measured by using GlcNAc6ST-2 detection system.
Step (B) of Example 4 was repeated, except that the sera of the patients diluted with PBS-T by a factor of 10 were used, instead of GlcNAc6ST-2 (*E. coli*). The amount of GlcNAc6ST-2 in the sera was determined by the standard curve (B) in Fig. 3. The value of an average + 2SD in healthy adult women was defined as a cut-off point, and it was determined whether the serum of each patient was positive or negative.

### Comparative Example 1

In the sera of 60 cases of the ovarian cancer patients and in the sera of 8 samples of the healthy adult women as tested in Example 5, CA125 which is known as a conventional marker for ovarian cancer was measured.
The measurement of CA125 was carried out by using a kit of ECLusys reagent CA125II (Roche Diagnostics), in accordance with the attached protocol.

### Comparative Example 2

In the sera of 60 cases of the ovarian cancer patients and in the sera of 8 samples of the healthy adult women as tested in Example 5, CA546 which is known as a conventional marker for ovarian cancer was measured.
The measurement of CA546 was carried out by using a kit of PLATE Kainos CA546 reagent (Kainos Laboratories, Inc.) in accordance with the attached protocol.

### Analytic Example 1

The 60 tested cases of ovarian cancer were classified into disease stages or histological types of ovarian cancer and positive rates obtained from Examples 4 and 5, and Comparative Example 1 and 2 were analyzed. The results are shown in Table 1.
In the early ovarian cancer of stages I and II, the positive rate detected using β3Gal-T5 detection system was 87.1%, and the positive rate detected by using GlcNAc6ST-2 detection system was 74.2%. It was possible to detect ovarian cancer at high rate in comparison with the positive rates detected using CA125 and CA546. Further, in the measurement by using β3Gal-T5 detection system and/or GlcNAc6ST-2 detection system, 96.8% of samples ware positive. Thus, it was possible to detect the early ovarian cancer patients at high rate, by combining the immunological methods of β3Gal-T5 and GlcNAc6ST-2.
Moreover, if ovarian cancers are classified into each of the histological types, the positive rate for mucinous adenocarcinoma was 100%, the positive rate for endometrioid adenocarcinoma was 84.6%, and the positive rate for clear cell adenocarcinoma was 87.5%, in accordance with the immunological method by using β3Gal-T5 detection system. These positive rates were higher than those obtained by CA125 or CA546. In particular, the immunological method using β3Gal-T5 detection system was able to detect the mucinous adenocarcinoma and clear cell adenocarcinoma at high rate, even thought these cancers are usually intractable and resistive to the chemotherapies.
In addition thereto, the positive rate for endometrioid ovarian cancer was 100% and the positive rate for clear cell ovarian cancer was 93.8% by combining the immunological methods of β3Gal-T5 detection system and GlcNAc6ST-2 detection system. Thus, it was able to detect these types of ovarian cancer at a high rate.

### Example 7: Measurement in sera of endometrial cancer patients by using β3Gal-T5 detection system

Sera of 30 cases of endometrial cancer patients and sera of 40 samples of healthy adult women, were measured using the β3Gal-T5 detection system. Among the endometrial cancer patients, 26 patients were surgically diagnosed as stage I to stage III. In one case among the 30 cases, a supervenience of cervical cancer was confirmed. In other 29 case, any cancer developments other than the endometrial cancer were not confirmed.

Step (C) of Example 4 was repeated, except that the sera of the endometrial cancer patients and healthy human diluted with PBS-T by a factor of 10 were added, instead of denatured β3Gal-T5 (*E. coli*). β3Gal-T5 values in the sera were determined by the standard curve (A) in Fig. 5. The results obtained from the 30 cases of the endometrial cancer patients are shown in Table 2. The value of an average + 2SD in healthy adult women was defined as a cut-off point, and it was determined whether the serum of the patient was positive or negative. In this case, the average value for healthy women was 2.1ng/mL and the cut-off point was 5.4ng/mL. Eighteen cases (60%) among the 30 case were positive.

### Example 8: Measurement in sera of endometrial cancer patients by using GlcNAc6ST-2 detection system

Sera of 30 cases of the endometrial cancer patients and sera of 40 samples of the healthy adult women as tested in Example 7, were measured by using GlcNAc6ST-2 detection system.
Step (D) of Example 4 was repeated, except that the sera of the patients diluted with PBS-T by a factor of 10 were added, instead of GlcNAc6ST-2 (*E. coli*)*.* GlcNAc6ST-2 values in the sera were determined by the standard curve (B) in Fig. 5. The results obtained from the 30 cases of the endometrial cancer patients were shown in Table 2. The value of an average + 2SD in healthy adult women was defined as a cut-off point, and it was determined whether the sera of the patient was positive or negative. In this case, the average value for healthy women was 2.0ng/mL and the cut-off point was 7.8ng/mL. Ten cases (33%) among the 30 case were positive.

### Comparative Example 3

In the sera of 9 cases of the endometrial cancer patients among 30 samples as tested in Example 7, CA125 which is known as a conventional marker for endometrial cancer was measured.
The measurement of CA125 was carried out by using a kit of ECLusys reagent CA125II (Roche Diagnostics) in accordance with the attached protocol. A measurement value 35U/mL or more was determined as positive. The results are shown in Table 2. 5 cases (38.5%) among 13 cases were positive.

### Comparative Example 4

In the sera of 19 cases of the endometrial cancer patients among the 30 cases as tested in Example 7, CA602 which is known as a conventional marker for endometrial cancer was measured.
The measurement of CA602 was carried out by using a kit of PLATE Kainos CA602 reagent (Kainos Laboratories, Inc.) in accordance with the attached protocol. A measurement value of 63U/mL or more was determined as positive. The results are shown in Table 2. 5 cases (26.3%) among 19 cases were positive.

### Comparative Example 5

In the sera of 23 cases of the endometrial cancer patients among the 30 cases as tested in Example 7, CA19-9 which is known as a marker for pancreas cancer and a marker for endometrial cancer was measured.
The measurement of CA19-9 was carried out by using a kit of ECLusys reagent CA19-9 II (Roche Diagnostics) in accordance with the attached protocol. A measurement value of 37U/mL or more was determined as positive. The results are shown in Table 2. 4 cases (16.7%) among 24 cases were positive.

**Table 2**

| Sample number | Disease stage | grade³⁾ | CA602 | CA125 | CA19-9 | GlcNAc6ST2 | B3Gal-T4/T5 |
|---|---|---|---|---|---|---|---|
| 1 | 1A | G1 | 4 | NT | 8 | **15.2** | 0 |
| 2 | 1B | G2 | 48 | NT | 22 | **262** | 0 |
| 3 | 2A | G1 | 9 | NT | 7 | 0 | **25.1** |
| 4 | 3C | G1 | NT²⁾ | 22 | **82** | **17.9** | **41.1** |
| 5 | 2A | G1 | 19 | **93** | 12 | **15.1** | **62.9** |
| 6 | 1B | G1 | **94** | NT | **88** | 0 | **49.9** |
| 7 | 3A | Carcinosarcoma | **614** | NT | 13 | **16.0** | 0 |
| 8 | 1B | G1 | 6 | NT | <1 | 0 | 0 |
| 9 | 1A | G1 | 25 | NT | 24 | 0 | 0 |
| 10 | 3C | G3 | NT | **830** | **1145** | 3.0 | 0 |
| 11 | 3A | G3 | NT | **81** | 13 | 0 | 0 |
| 12 | 1B | G3 | NT | 28 | 22 | **23.6** | **21.3** |
| 13 | 1B | G1 | 21 | 26 | 19 | 0 | 0 |
| 14 | 1B | G1 | 15 | NT | NT | **9.8** | **26.4** |
| 15 | 1A | G1 | 8 | NT | 7 | 4.0 | **24.7** |
| 16 | 1B | G1 | NT | NT | NT | 6.4 | 0 |
| 17 | 1A | G1 | NT | 4 | 28 | 0 | 0 |
| 18 | 2A | G1 | 20 | NT | 28 | 3.2 | **31.8** |
| 19 | 1B | G1 | 9 | NT | 19 | 6.0 | **32.3** |
| 20 | 1C | G2 | 22 | 22 | <1 | 0 | 0 |
| 21 | 1A | G1 | 29 | NT | 7 | 5.8 | **24.7** |
| 22 | 1B | G2 | 12 | NT | 8 | 6.3 | **20.9** |
| 23 | 1C | G1 | **81** | NT | **100** | **28.6** | **29.7** |
| 24 | 2A | G1 | NT | 15 | <1 | 5.1 | **9.2** |
| 25 | 3A¹⁾ | G1 | **92** | NT | NT | 0 | **21.8** |
| 26 | 3A | G3 | **409** | NT | 15 | 0 | 0 |
| 27 | | | NT | **214** | 19 | 0 | **13.1** |
| 28 | | | NT | **84** | NT | **63.3** | **22.9** |
| 29 | | | NT | 11 | NT | 0.4 | **9.0** |
| 30 | | | NT | 25 | NT | **28.8** | **11.2** |

| | | | | | | | |
|---|---|---|---|---|---|---|---|
| ¹⁾ : complicated with cervical cancer ²⁾ : NT: not tested ³⁾ : Grade represents a degree of histodifferentiation (i.e., grade of malignancy of endometrial cancer). G1 (grade 1) indicates a well-differentiated form, and is a type which is similar to the histologically-normal gland structure. On the other hand, G3 (grade 3) indicates a poorly-differentiated form, and is a type which shows a random propagation. | | | | | | | |

### Analytic Example 2

The results shown in Table 2 were reclassified into the positive rates obtained by each of markers, as shown in Table 3.

**Table 3**

| **Measurement results in the sera of the endometrial cancer patients** | | | |
|---|---|---|---|
| **Marker** | **Number of samples (a)** | **Number of positive samples (b)** | **Positive rate 5 % (b/a x 100)** |
| CA602 | 19 | 5 | 26.3 |
| CA125 | 13 | 5 | 38.5 |
| CA19-9 | 24 | 4 | 16.7 |
| GlcNAcST2 | 30 | 10 | 33.3 |
| *β* 3Gal-T4/T5 | 30 | 18 | 60.0 |
| GlcNAcST2 + *β* 3Gal-T4/T5 | 30 | 21 | 70.0 |

The positive rates for each of CA602, CA125, and CA19-9, known as conventional markers for the endometrial cancer, were 26.3%, 38.5%, and 16.7%, respectively. On the other hand, the positive rate for β3Gal-T5 detection system was 60 %, and the high positive rate was confirmed. Further, the positive rate for GlcNAc6ST-2 detection system was 33.3%, which was higher than the positive rates for CA602 and CA19-9, and lower than the positive rate for CA125. However, it was revealed that the GlcNAc6ST-2 detection system can effectively detect the endometrial cancer which was not detected by the conventional markers, as shown in the sample numbers of 1, 2, and 12 of Table 2. Further, in the measurement using the β3Gal-T5 detection system and/or GlcNAc6ST-2 detection system, 70% of samples ware positive. Thus, it was possible to detect the endometrial cancer at high rates, by combining the immunological methods of the β3Gal-T5 detection system and the GlcNAc6ST-2 detection system.

### Example 9

Sera of 6 cases of the endometrial cancer patients were measured by using the β3Gal-T5 detection system. 6 cases from the present invention and 30 cases measured in Example 7 were divided into the disease stage I and disease stages II to IV. The positive rates for each of the disease stage I and disease stages II to IV are shown in Table 4.

**Table 4**

| **Positive rate for the *β* 3Gal-T5 detection system according to the stage of disease** | | | |
|---|---|---|---|
| **Disease stages** | **Number of samples (a)** | **Number of positive samples (b)** | **Positive rate % (b/a x 100)** |
| **Stage I** | **21** | **12** | **57.1** |
| **Stages II to IV** | **15** | **11** | **73.3** |

As shown in Table 4, the positive rate for β3Gal-T5 detection system in the disease stage I of early endometrial cancer was 57.1%, and it was possible to detect the endometrial cancer at high rates, even in the early stage of disease.

### INDUSTRIAL APPLICABILITY

In accordance with the detecting method and the detecting kit for ovarian cancer according to the invention, it is possible to detect with high rates of success ovarian cancer or endometrial cancer.
Although the present invention has been described with reference to specific embodiments, various changes and modifications obvious to those skilled in the art are possible without departing from the scope of the appended claims.

## Claims

1. An in vitro method for detecting ovarian cancer **characterized in that** β1,3-galactosyltransferase-5 and/or β1,3-galactosyltransferase-4 are detected.

2. A in vitro method for detecting endometrial cancer **characterized in that** ß1,3-galactosyltransferase-5 and/or ß1,3-galactosyltransferase-4 in blood, serum, or plasma are detected.

3. An in vitro method for detecting ovarian cancer **characterized in that** GlcNAc 6-O-sulfotransferase-2 in blood, serum, or plasma is detected.

4. An in vitro method for detecting endometrial cancer **characterized in that** GlcNAc 6-O-sulfotransferase-2 is detected.

5. An in vitro method for detecting ovarian cancer **characterized in that** ß1,3-galactosyltransferase-5 and/or ß1,3-galactosyltransferase-4, and GlcNAc 6-O-sulfotransferase-2 are detected.

6. A An in vitro method for detecting endometrial cancer **characterized in that** ß1,3-galactosyltransferase-5 and/or ß1,3-galactosyltransferase-4, and GlcNAc 6-O-sulfotransferase-2 are detected.

7. The method according to any of claims 1 , 2, 5 and 6, **characterized in that** an antibody specifically binding to ß1,3-galactosyltransferase-5 and ß1,3-galactosyltransferase-4 is used.

8. The method according to any of claims 3 to 6, **characterized in that** an antibody specifically binding to GlcNAc 6-O-sulfotransferase-2 is used.

9. Use of a kit for detecting ovarian cancer in vitro, wherein the kit contains an antibody or a fragment thereof having specific binding ability to ß1,3-galactosyltransferase-5 and ß1,3-galactosyltransferase-4.

10. Use of a kit for detecting endometrial cancer in vitro by detecting ß1,3-galactosyltransferase-5 and/or ß1,3-galactosyltransferase-4 in blood, serum or plasma, wherein the kit contains an antibody or a fragment thereof having specific binding ability to to ß1,3-galactosyltransferase-5 and ß1,3-galactosyltransferase-4.

11. Use of a kit for detecting ovarian cancer in vitro by detecting GlcNAc 6-O-sulfotransferase-2 in blood, serum or plasma, wherein the kit contains an antibody or a fragment thereof having specific binding ability to GlcNAc 6-O-sulfotransferase-2.

12. Use of a kit for detecting endometrial cancer in vitro, wherein the kit contains an antibody or a fragment thereof having specific binding ability to GlcNAc 6-O-sulfotransferase-2.

13. A kit for detecting ovarian cancer **characterized by** containing an antibody or a fragment thereof having specific binding ability to ß1,3-galactosyltransferase-5 and ß1,3-galactosyltransferase-4, and an antibody or a fragment thereof having specific binding ability to GlcNAc 6-O-sulfotransferase-2.

14. A kit for detecting endometrial cancer **characterized by** containing an antibody or a fragment thereof having specific binding ability to ß1,3-galactosyltransferase-S and ß1,3-galactosyltransferase-4, and an antibody or a fragment thereof having specific binding ability to GlcNAc 6-O-sulfotransferase-2,

## Patentansprüche

1. In vitro Verfahren zum Detektieren von Eierstockkrebs, **dadurch gekennzeichnet, dass** β1,3-Galactosyltransferase-5 und/oder β1,3-Galactosyltransferase-4 detektiert werden.

2. In vitro Verfahren zum Detektieren von Endometriumkrebs, **dadurch gekennzeichnet, dass** β1,3-Galactosyltransferase-5 und/oder β1,3-Galactosyltransferase-4 in Blut, Serum oder Plasma detektiert werden.

3. In vitro Verfahren zum Detektieren von Eierstockkrebs, **dadurch gekennzeichnet, dass** GlcNAc 6-O-Sulfotransferase-2 in Blut, Serum oder Plasma detektiert wird.

4. In vitro Verfahren zum Detektieren von Endometriumkrebs, **dadurch gekennzeichnet, dass** GlcNAc 6-O-Sulfotransferase-2 detektiert wird.

5. In vitro Verfahren zum Detektieren von Eierstockkrebs, **dadurch gekennzeichnet, dass** β1,3-Galactosyltransferase-5 und/oder β1,3-Galactosyltransferase-4 und GlcNAc 6-O-Sulfotransferase-2 detektiert werden.

6. In vitro Verfahren zum Detektieren von Endometriumkrebs, **dadurch gekennzeichnet, dass** β1,3-Galactosyltransferase-5 und/oder β1,3-Galactosyltransferase-4 und GlcNAc 6-O-Sulfotransferase-2 detektiert werden.

7. Verfahren nach einem der Ansprüche 1, 2, 5 und 6, **dadurch gekennzeichnet, dass** ein Antikörper, welcher spezifisch an β1,3-Galactosyltransferase-5 und β1,3-Galactosyltransferase-4 bindet, verwendet wird.

8. Verfahren nach einem der Ansprüche 3 bis 6, **dadurch gekennzeichnet, dass** ein Antikörper, welcher spezifisch an GlcNAc 6-O-Sulfotransferase-2 bindet, verwendet wird.

9. Verwendung eines Kits zum Detektieren von Eierstockkrebs in vitro, wobei das Kit einen Antikörper oder ein Fragment davon, welcher spezifische Bindungsfähigkeit an β1,3-Galactosyltransferase-5 und β1,3-Galactosyltransferase-4 aufweist, enthält.

10. Verwendung eines Kits zum Detektieren von Endometriumkrebs in vitro durch Detektieren von β1,3-Galactosyltransferase-5 und/oder β1,3-Galactosyltransferase-4 in Blut, Serum oder Plasma, wobei das Kit einen Antikörper oder ein Fragment davon, welcher spezifische Bindungsfähigkeit an β1,3-Galactosyltransferase-5 und β1,3-Galactosyltransferase-4 aufweist, enthält.

11. Verwendung eines Kits zum Detektieren von Eierstockkrebs in vitro durch Detektieren von GlcNAc 6-O-Sulfotransferase-2 in Blut, Serum oder Plasma, wobei das Kit einen Antikörper oder ein Fragment davon, welcher spezifische Bindungsfähigkeit an GlcNAc 6-O-Sulfotransferase-2 aufweist, enthält.

12. Verwendung eines Kits zum Detektieren von Endometriumkrebs in vitro, wobei das Kit einen Antikörper oder ein Fragment davon, welcher spezifische Bindungsfähigkeit an GlcNAc 6-O-Sulfotransferase-2 aufweist, enthält.

13. Kit zum Detektieren von Eierstockkrebs, **dadurch gekennzeichnet, dass** es einen Antikörper oder ein Fragment davon, welcher spezifische Bindungsfähigkeit an β1,3-Galactosyltransferase-5 und β1,3-Galactosyltransferase-4 aufweist, und einen Antikörper oder ein Fragment davon, welcher spezifische Bindungsfähigkeit an GlcNAc 6-O-Sulfotransferase-2 aufweist, enthält.

14. Kit zum Detektieren von Endometriumkrebs, **dadurch gekennzeichnet, dass** es einen Antikörper oder ein Fragment davon, welcher spezifische Bindungsfähigkeit an β1,3-Galactosyltransferase-5 und β1,3-Galactosyltransferase-4 aufweist, und einen Antikörper oder ein Fragment davon, welcher spezifische Bindungsfähigkeit an GlcNAc 6-O-Sulfotransferase-2 aufweist, enthält.

## Revendications

1. Procédé *in vitro* de détection d'un cancer ovarien **caractérisé en ce que** de la β1,3-galactosyltransférase-5 et/ou de la β1,3-galactosyltransférase-4 sont détectées.

2. Procédé *in vitro* de détection d'un cancer endométrial **caractérisé en ce que** de la β1,3-galactosyltransférase-5 et/ou de la β1,3-galactosyltransférase-4 dans le sang, le sérum ou le plasma sont détectées.

3. Procédé *in vitro* de détection d'un cancer ovarien **caractérisé en ce que** de la GlcNAc 6-O-sulfotransférase-2 dans le sang, le sérum ou le plasma est détectée.

4. Procédé *in vitro* de détection d'un cancer endométrial **caractérisé en ce que** de la GlcNAc 6-O-sulfotransférase-2 est détectée.

5. Procédé *in vitro* de détection d'un cancer ovarien **caractérisé en ce que** de la β1,3-galactosyltransférase-5 et/ou de la β1,3-galactosyltransférase-4, et de la GlcNAc 6-O-sulfotransférase-2 sont détectées.

6. Procédé *in vitro* de détection d'un cancer endométrial **caractérisé en ce que** de la β1,3-galactosyltransférase-5 et/ou de la β1,3-galactosyltransférase-4, et de la GlcNAc 6-O-sulfotransférase-2 sont détectées.

7. Procédé selon l'une quelconque des revendications 1, 2, 5 et 6, **caractérisé en ce qu'**un anticorps se liant spécifiquement à la β1,3-galactosyltransférase-5 et à la β1,3-galactosyltransférase-4 est utilisé.

8. Procédé selon l'une quelconque des revendications 3 à 6, **caractérisé en ce qu'**un anticorps se liant spécifiquement à la GlcNAc 6-O-sulfotransférase-2 est utilisé.

9. Utilisation d'un kit pour la détection d'un cancer ovarien *in vitro,* où le kit contient un anticorps ou un fragment de celui-ci présentant une capacité de liaison spécifique à la β1,3-galactosyltransférase-5 et à la β1,3-galactosyltransférase-4.

10. Utilisation d'un kit pour la détection d'un cancer endométrial *in vitro* par la détection de β1,3-galactosyltransférase-5 et/ou de β1,3-galactosyltransférase-4 dans le sang, le sérum ou le plasma, où le kit contient un anticorps ou un fragment de celui-ci présentant une capacité de liaison spécifique à la β1,3-galactosyltransférase-5 et à la β1,3-galactosyltransférase-4.

11. Utilisation d'un kit pour la détection d'un cancer ovarien *in vitro* par la détection de GlcNAc 6-O-sulfotransférase-2 dans le sang, le sérum ou le plasma, où le kit contient un anticorps ou un fragment de celui-ci présentant une capacité de liaison spécifique à la GlcNAc 6-O-sulfotransférase-2.

12. Utilisation d'un kit pour la détection d'un cancer endométrial *in vitro,* où le kit contient un anticorps ou un fragment de celui-ci présentant une capacité de liaison spécifique à la GlcNAc 6-O-sulfotransférase-2.

13. Kit pour la détection d'un cancer ovarien **caractérisé en ce qu'**il contient un anticorps ou un fragment de celui-ci présentant une capacité de liaison spécifique à la β1,3-galactosyltransférase-5 et à la β1,3-galactosyltransférase-4, et un anticorps ou un fragment de celui-ci présentant une capacité de liaison spécifique à la GlcNAc 6-O-sulfotransférase-2.

14. Kit pour la détection d'un cancer endométrial **caractérisé en ce qu'**il contient un anticorps ou un fragment de celui-ci présentant une capacité de liaison spécifique à la β1,3-galactosyltransférase-5 et à la β1,3-galactosyltransférase-4, et un anticorps ou un fragment de celui-ci présentant une capacité de liaison spécifique à la GlcNAc 6-O-sulfotransférase-2.
